# EUROPEAN PATENT SPECIFICATION

(11) **EP 4 204 585 B1**
(45) Date of publication and mention of the grant of the patent: **31.12.2025**
(21) Application number: 21860757.0
(22) Date of filing: 25.08.2021
(51) Int. Cl.: C12Q 1/6858, C12Q 1/6851, C12Q 1/70

(54) **ULTRASENSITIVE RNA QUANTIFICATION USING NANOPORES**
QUANTIFIZIERUNG VON ULTRAEMPFINDLICHER RNA MIT NANOPOREN
QUANTIFICATION ULTRASENSIBLE D'ARN À L'AIDE DE NANOPORES

(30) Priority: 25.08.2020 US 202063069826 P
(43) Date of publication of application: 05.07.2023
(73) Proprietor: TECHNION RESEARCH & DEVELOPMENT FOUNDATION LIMITED, 3200004 Haifa (IL)
(72) Inventor: MELLER, Amit, 3570349 Haifa (IL); ROZEVSKY, Yana, 2623014 Kiryat Motzkin (IL); GILBOA, Tal, 3655104 Kiryat Tivon (IL)
(74) Representative: Boult Wade Tennant LLP
(86) International application number: PCT/IL2021/051045
(87) International publication number: WO 2022/044012

(56) References cited:
- WO-A1-2016/149021
- WO-A2-2018/069484
- US-A1- 2019 211 394
- BYRNE ASHLEY ET AL: "Depletion of Hemoglobin Transcripts and Long-Read Sequencing Improves the Transcriptome Annotation of the Polar Bear (Ursus maritimus)", FRONTIERS IN GENETICS, vol. 10, no. 643, 19 July 2019 (2019-07-19), pages 1 - 12, XP055910431, DOI: 10.3389/fgene.2019.00643
- HASHIMSHONY, T. ET AL.: "CEL-Seq: single- cell RNA-Seq by multiplexed linear amplification", CELL REPORTS, vol. 2, no. 3, 27 September 2012 (2012-09-27), pages 666 - 673, XP055111758, DOI: 10.1016/j.celrep.2012.08.003
- BYRNE ASHLEY, SUPPLE MEGAN A., VOLDEN ROGER, LAIDRE KRISTIN L., SHAPIRO BETH, VOLLMERS CHRISTOPHER: "Depletion of Hemoglobin Transcripts and Long-Read Sequencing Improves the Transcriptome Annotation of the Polar Bear (Ursus maritimus)", FRONTIERS IN GENETICS, vol. 10, no. 643, pages 1 - 12, XP055910431, DOI: 10.3389/fgene.2019.00643
- HARRIET D. GLIDDON; JETHRO A. HERBERG; MICHAEL LEVIN; MYRSINI KAFOROU: "Genome‐wide host RNA signatures of infectious diseases: discovery and clinical translation", IMMUNOLOGY, vol. 153, no. 2, 24 October 2017 (2017-10-24), GB , pages 171 - 178, XP071276840, ISSN: 0019-2805, DOI: 10.1111/imm.12841
- LIU, L. ET AL.: "Simultaneous Quantification of Multiple Cancer Biomarkers in Blood Samples through DNA-Assisted Nanopore Sensing", ANGEWANDTE CHEMIE, vol. 57, no. 37, 10 September 2018 (2018-09-10), pages 11882 - 11887, XP055910446
- KIDAN LEE, PARK KYEONG-BEOM, KIM HYUNG-JUN, YU JAE-SEOK, CHAE HONGSIK, KIM HYUN-MI, KIM KI-BUM: "Recent Progress in Solid-State Nanopores", ADVANCED MATERIALS, vol. 30, no. 42, DE , pages 1704680, XP055566192, ISSN: 0935-9648, DOI: 10.1002/adma.201704680

## Description

### CROSS-REFERENCE TO RELATED APPLICATIONS

This application claims the benefit of priority of U.S. Provisional Patent Application No. 63/069,826 filed August 25, 2020.

### FIELD OF INVENTION

The present invention is in the field of nanopore biosensing.

### BACKGROUND OF THE INVENTION

The ability to sense and digitally count individual RNA and mRNA biomarker molecules holds a great potential for early diagnosis of a wide range of diseases including the recent severe acute respiratory syndrome coronavirus 2 (SARS-CoV-2) and cancer. The 'gold-standard' method for RNA molecule quantification in clinical samples relies on reverse transcription, followed by a massive non-linear amplification of the DNA molecules using Reverse Transcription quantitative Real-Time Polymerase Chain Reaction (RT qRT-PCR). RT qRT-PCR relies on bulk fluorescence signal measurement during the annealing step of each PCR amplification cycle. An offline analysis process is used to empirically determine a crossing threshold value (*C_{T}*), indicating the earliest cycle at which the PCR product is reliably detected above the background noise in the exponential amplification regime. Using a calibration curve of known initial total RNA amounts, the *C_{T}* values are then used to assess the initial quantity of the analyzed transcript. Notably, RT qRT-PCR is fundamentally limited by a baseline noise level, which relies on gene-specific amplification relative to the amplification of chosen housekeeping genes. Further, low abundancy of the target RNA in the clinical sample has been shown to evade sensing, leading to false negative diagnosis, whereas excessive PCR amplification may deteriorate its specificity.

Cancer screening is also severely limited in its ability to quickly, cheaply and accurately detect those subjects with as yet undiagnosed cancer. For example, current colorectal cancer (CRC) screening techniques are based on the fecal occult blood test (FOBT), flexible sigmoidoscopy and colonoscopy. The FOBT is based on the presence of occult blood in the stool samples. This technique is cheap and non-invasive but has low sensitive with large numbers of false positives. Flexible sigmoidoscopy is an endoscopic examination method used for the majority of adenomas and cancers, which are located in the distal colon. This method is invasive, more sensitive (60-70% of that achieved by colonoscopy), but costly and uncomfortable for the patients. Colonoscopy is the gold standard procedure for removing adenomas and lesions of cancer. In some countries such as Poland and Germany and most commonly in the USA, colonoscopy is used as a primary CRC screening method. In others, individuals with positive result from other screening CRC methods need to be re-screen by colonoscopy. Colonoscopy is an invasive method with high sensitivity; however, it is costly, and thus offers limited availability to lower socioeconomical populations.

In recent years, an enormous effort has been made to establish prognostic tools for CRC, in particular there has been a search for biomarkers, in order to increase the sensitivity of pre-screening and provide information on the outcome irrespective of the treatment used. This effort has led to a greater understanding of the biological pathways associated with CRC and has pointed toward the epidermal growth factor receptor (EGFR) as a critical mechanism. Activation of EGFR stimulates key processes involved in tumor growth and progression via two main pathways: the KRAS-RAF-mitogen-activated protein kinase, responsible for gene transcription, cell cycle progression and cell proliferation; and the lipid kinase phosphatidylinositol 3-kinase (PI3K), which promotes Akt-mammalian target of rapapycin (mTOR) activation responsible for anti-apoptosis signals.

Despite broad consensus favoring the introduction of KRAS testing in clinical practice as a mean to select patients before drug administration the current available methods remain limited and insufficient. Currently the most widely applied method to for assessing KRAS mutations is direct dideoxy DNA sequencing and PCR based assays, which have relatively low sensitivity because mutant alleles have to be present in at least 10%-20% of cells to be reproducibly detected.

Solid-state Nanopores (ss-NPs) have recently emerged as label-free single-molecule sensing platforms for nucleic acids and proteins. Relative quantification of nucleic acid species in a sample has been shown to be feasible using ss-NPs, only limited by the fundamental Poisson counting statistics. To date, however, the quantification of ultra-low RNAs from biological or clinical samples, has been challenging. This is attributed to the complexity associated with interpreting the signals obtained from highly heterogenous biological specimens. A solution that addresses this limitation and allows for ultrasensitive quantitation of specific RNAs is greatly needed. WO 2018/069484 discloses a method for detecting and/or quantifying mRNA or cDNA using a nanopore. Byrne et al., Frontiers in genetics (19.07.2019) discloses detection of rare transcripts using a protocol including RNAse and proteinase treatment followed by nanopore sequencing.

### SUMMARY OF THE INVENTION

The present invention provides methods for quantifying a species of RNA within a sample as well as methods of diagnosing a disease in a subject.

According to a first aspect, there is provided a method for quantifying a first species of RNA within a sample, the method comprising:
a. receiving a sample comprising RNA and substantially devoid of DNA polymers;
b. contacting the sample with a first primer that hybridizes to the first species of RNA under conditions sufficient for reverse transcription (RT) of the first species of RNA to cDNA, thereby producing a first cDNA strand complementary to at least a portion of the species of RNA and comprising the first primer;
c. treating the sample with an RNAse enzyme thereby producing a sample comprising the first cDNA strand and substantially devoid of RNA polymers;
d. contacting the sample with a second primer that hybridizes to the first cDNA strand and performing 1-5 cycles of amplification to produce amplification products with a first termini that is identical to a sequence of the first primer and a second termini that is a reverse complement to a sequence of the second primer or with a first termini that is a reverse complement of a sequence of the first primer and a second termini that is identical to a sequence of the second primer;
e. treating the sample with a proteinase enzyme, wherein the enzyme comprises a net positive charge, to produce a solution substantially devoid of polypeptides other than the proteinase;
f. depositing the solution within a first reservoir of a nanopore containing apparatus and passing the amplification products through the nanopore by running electrical current from the first reservoir to a second reservoir via the nanopore; and
g. identifying an amplification product derived from the first RNA species as it passes through the nanopore by the amplification product's dwell time within the nanopore;
wherein said method is devoid of a washing or isolation step after step (a), thereby quantifying a species of RNA within a sample.

According to another aspect, there is provided a method of diagnosing a disease in a subject in need thereof, the method comprising performing a method of the invention, wherein the sample is from the subject, the first RNA species is an mRNA of a gene associated with the disease, and detection of the first RNA species in the sample indicates the subject suffers from the disease.

According to some embodiments, the first RNA species is a lowly abundant RNA species in the sample.

According to some embodiments, the receiving comprises receiving a sample of isolated RNA.

According to some embodiments, the receiving comprises receiving a cell lysate contacted with a DNAse enzyme.

According to some embodiments, the method further comprises receiving a cellular lysate and contacting the lysate with a DNAse enzyme to produce a sample comprising RNA and substantially devoid of DNA.

According to some embodiments, (b) comprises contacting the sample with a reverse transcriptase and DNA nucleotides.

According to some embodiments, the first primer, the second primer or both are DNA primers.

According to some embodiments, the step (c) occurs before the step (d).

According to some embodiments, the step (c) occurs after the step (d) and the RNAse treatment produces a sample comprising the first cDNA strand and the amplification products are substantially devoid of RNA.

According to some embodiments, an enzyme carrying a net positive charge is an enzyme that when in a solution through which electrical current is passed migrates towards a negative pole.

According to some embodiments, the proteinase is proteinase K.

According to some embodiments, the treating with a proteinase is under conditions sufficient for degradation of polypeptides to single amino acids.

According to some embodiments, the conditions are conditions sufficient for protection of the proteinase from autolysis.

According to some embodiments, the amplification product is at least 20 nucleotides shorter than the first cDNA strand.

According to some embodiments, the first primer, the second primer or both is specific to the RNA species, 100% complementary to the RNA species or both.

According to some embodiments, the method comprises a single cycle of amplification thereby producing on average a single amplification product for each molecule of the species of RNA.

According to some embodiments, the amplification product is not larger than 10,000 nucleotides.

According to some embodiments, the amplification does not comprises integrating a detectable moiety into the amplification products.

According to some embodiments, the identifying comprises detecting a change in electrical current through the nanopore.

According to some embodiments, the identification by dwell time comprise measuring duration of the change in electrical current, and wherein the duration is proportional to the length of the amplification product.

According to some embodiments, identifying the amplification product derived from the RNA comprises differentiating the amplification product derived from the RNA from at least one of a free DNA nucleotide, a free RNA nucleotide, a free amino acid, a first cDNA strand and an off target amplification product by dwell time within the nanopore.

According to some embodiments, the method further comprises in step (b) contacting the sample with a third primer that hybridizes to a second species of RNA, thereby producing a first cDNA strand complementary to at least a portion of the second species of RNA and comprising the second primer; and further comprising in step (d) contacting the sample with a fourth primer that hybridizes to the first cDNA strand complementary to at least a portion of the second species of RNA to produce amplification products with a first termini that is identical to a sequence of the third primer and a second termini that is a reverse complement to a sequence of the fourth primer or with a first termini that is a reverse complement of a sequence of the third primer and a second termini that is identical to a sequence of the fourth primer.

According to some embodiments, amplification products derived from the first species and amplification products derived from the second species differ in length by at least 20 nucleotides and are differentiated by a difference in dwell time.

According to some embodiments, the amplification products derived from the first species and the amplification products derived from the second species differ in length by at least 100 nucleotides.

According to some embodiments, the method comprises quantifying at least three species of RNA within the sample, wherein each amplification product derived from an RNA species is at least 20 nucleotides in length different than any amplification product derived from a different RNA species.

According to some embodiments, the first species of RNA comprises a point mutation and the method further comprises:
a. contacting the amplification products with a first probe perfectly complementary to the amplification products and comprising a terminal nucleotide complementary to the point mutation and a second probe perfectly complementary to the amplification products directly adjacent to the point mutation and not complementary to the same region as the first probe;
b. ligating the first probe and the second probe to produce a ligated product, such that the ligated product comprises a difference in length from the amplification products, the first probe and the second probe of at least 15 nucleotides; and
c. identifying the ligated product as it passes through the nanopore.

According to some embodiments, the second probe comprises a detectable moiety and the identifying comprises detecting the detectable moiety as it passes through the nanopore; or wherein the second probe does not comprise a detectable moiety and the identification by dwell time comprise measuring duration of a change in electrical current, and wherein the duration is proportional to the length of a nucleic acid molecule translocating through the nanopore, allowing for distinguishing the ligated product from the amplification product.

According to some embodiments, the method is devoid of a washing or isolation step after step (a).

According to some embodiments, the first species of RNA is RNA of a target gene.

According to some embodiments, the second species of RNA is RNA of a control gene.

According to some embodiments, the control gene is selected from glucose-6-phosphate dehydrogenase (G6PDH) and Ribonuclease P/MRP subunit P30 (RPP30).

According to some embodiments, the first species of RNA is RNA of a disease-associated gene.

According to some embodiments, expression of the gene in the sample is indicative of the presence of the disease.

According to some embodiments, detection of the first RNA species above a predetermined threshold indicates the subject suffers from the disease.

According to some embodiments, the disease is an infectious disease and the first species of RNA is an RNA of an infectious agent.

According to some embodiments, the second RNA species is an RNA of a host cell infected by the infectious agent.

According to some embodiments, the infectious disease is SARS-CoV-2, and the first RNA species is an RNA-dependent RNA polymerase (RdRP) gene mRNA.

According to some embodiments, the disease is characterized by the presence of a mutation and the first species of RNA is an RNA comprising the mutation.

According to some embodiments, the disease is a proliferative disease and the mutation is pro-proliferative or antiapoptotic.

According to some embodiments, the method further comprises administering a therapeutic agent that treats the disease to a subject diagnosed with the disease.

### BRIEF DESCRIPTION OF THE DRAWINGS

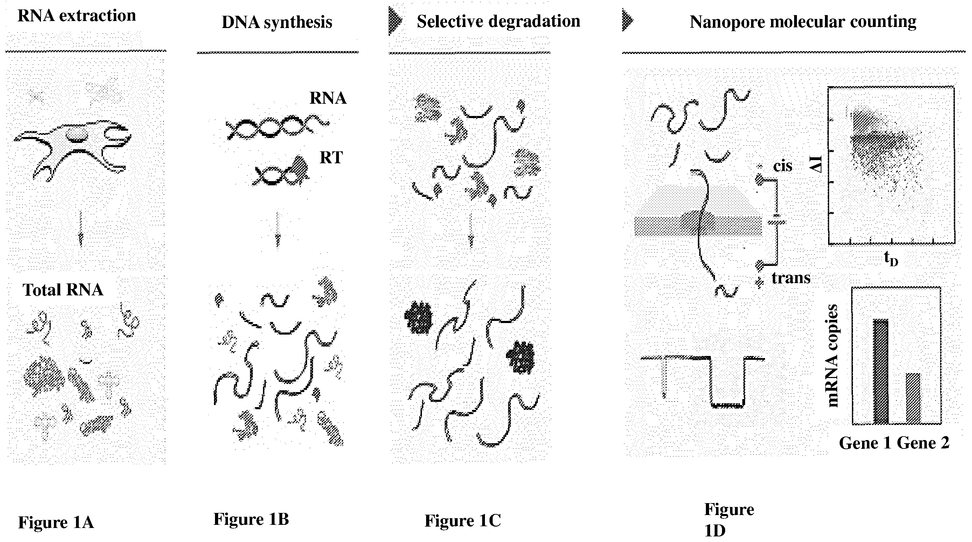

**Figures 1A****-D: Single-molecule mRNA quantification using enzymatic digestion followed by nanopore analysis.** Schematic illustration of the sample preparation steps, nanopore sensing and analysis of the results. The method involves four main steps: (**1A**) total RNA is extracted from whole cells, treated by DNaseI and further purified. **(1B)** Transcripts of interest are converted to cDNAs of specific lengths by reverse transcription and are amplified by PCR using a minimal number of cycles. **(1C)** Next, the sample is subjected to a two-step enzymatic digestion using RNase 1 and Proteinase K to remove off-target molecules. (**1D**) The sample is then introduced directly to a solid-state nanopore and each translocation event is analyzed individually to extract its electrical characteristics. Signal classification is used to cluster the molecules and the gene expression level of each transcript is estimated accordingly.

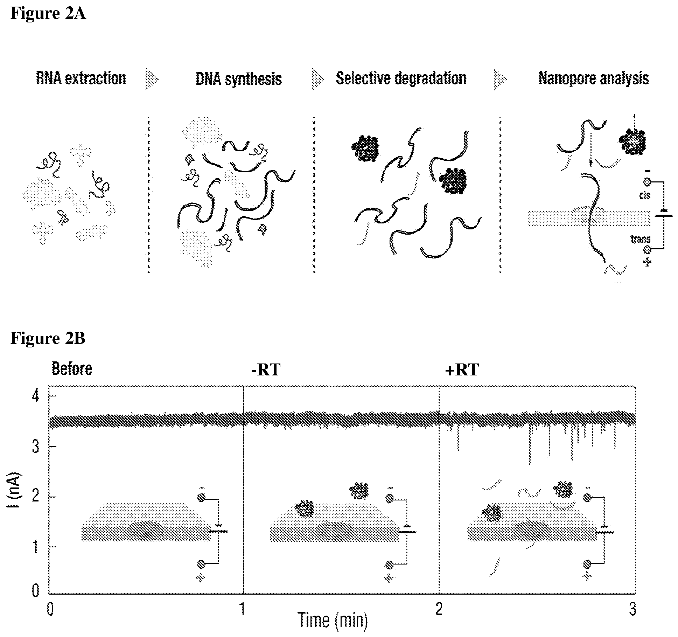

**Figures 2A****-G: (2A)** Three consecutive biochemical steps prepare mRNA (or RNA) molecules for nanopore analysis. i) RNA is extracted from cells or clinical samples. ii) Transcripts of interest are converted to cDNAs of specific lengths by reverse transcription followed by second strand DNA synthesis and optional 2 to 16-fold amplification. iii) The sample is subjected to digestion using RNase 1 and ProK to remove off-target molecules. **(2B)** An experimental validation of the RNA sensing method: 50 ng of DNaseI-treated total RNA, extracted from cell line 2, was subjected to reverse transcription in the presence of the RT enzyme ('+RT') or without it ('-RT'), using gene specific primers. Panels (left to right) show the nanopore ion current trace before addition of the analytes, addition of -RT sample, and addition of the +RT sample. **(2C)** Each DNA translocation event is analyzed to extract its electrical characteristics. A Gaussian Mixture Model (GMM) is used to cluster events and classify the molecules to produce a relative gene expression result. **(2D)** Gel electrophoresis of purified GOI1, GOI2, and RG (G6PDH) amplicons. Purified cDNA fragments for each gene were prepared using 50 ng of total RNA derived from cell line 2, and treated with DNase I. In each case, the samples underwent gene-specific cDNA synthesis, were amplified by PCR and treated with RNase I. Then, the samples were further purified using the QIAquick PCR purification kit (Qiagen) according to the manufacturer's instructions. Negative control samples (-RT) were subjected to the same preparation procedure without the RT enzyme. The cDNA samples were separated in 4% PAGE, stained with SYBR Gold, and imaged by GelDoc EZ (BioRad). **(2E)** Ionic current after addition of a -RT sample to the nanopore. No non-specific translocation events occur during 10 minutes of continuous recording at 300 mV bias, after addition of a -RT sample. The nanopore diameter is ~4 nm. (**2F-G**) Comparison of DNA translocation events with and without purification. GOI1 cDNA samples were prepared and amplified by 15 PCR cycles. Samples were either **(2F)** subjected to enzymatic digestion following the purification-free method and then diluted 1000-fold or **(2G)** purified using a PCR clean-up kit. Each sample was introduced into a separate nanopore at 300 mV bias (conductance ~9 nS, corresponding to ~4 nm diameter). Typical concatenated events are shown at the top. An event diagram and a histogram of the blockage amplitude are shown in the middle (individual events marked as dots), and a histogram of the event dwell times is shown at the bottom.

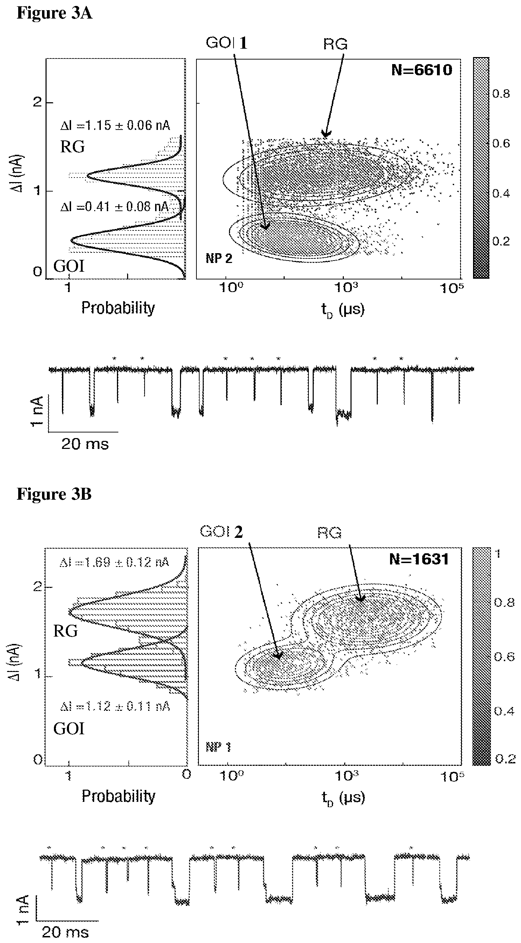

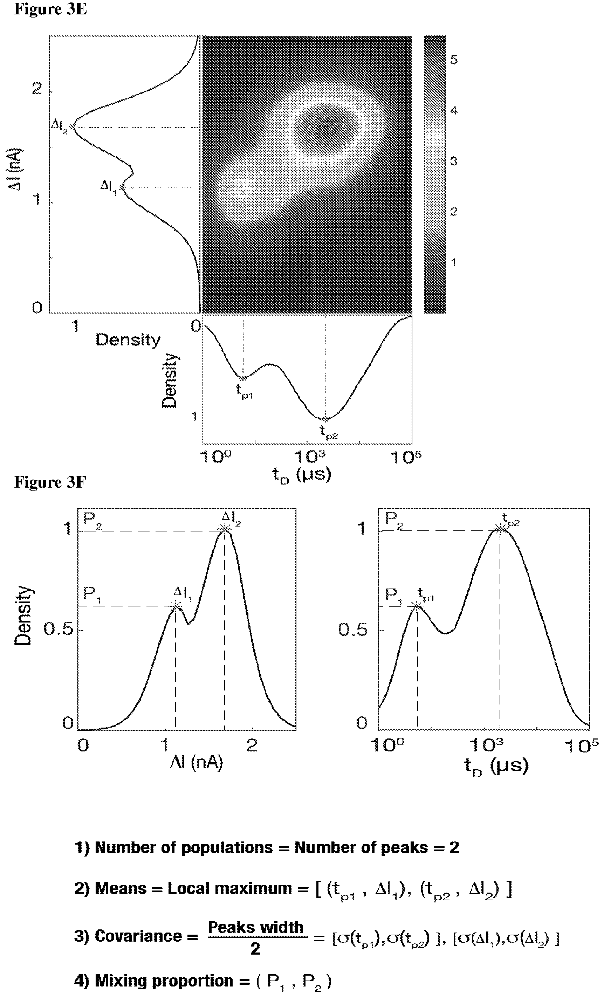

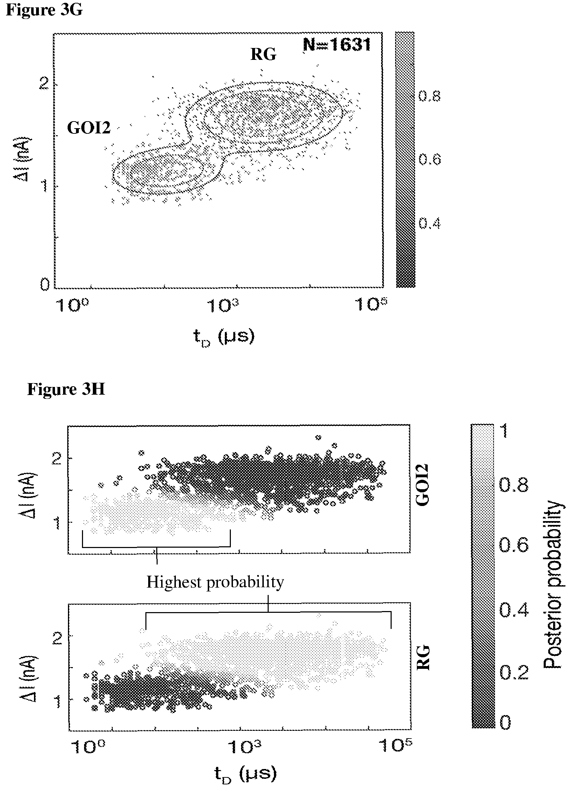

**Figures 3A****-H: (3A-D)** Validation of GMM classification of multiple genes by ssNPs. Upper panel: scatter plots and the associated histograms obtained **(3A)** for a mixture of 0.5 nM GOI1 (360 bp) and 1 nM RG (1231 bp) cDNAs, and **(3B)** for a mixture of 0.5 nM GOI2 (123 bp) and 1 nM RG cDNAs. The conductance of the pores was 15.2 nS and 22.7 nS. The translocation events of each sample mixture were classified using a two-dimensional GMM algorithm. The fitted Gaussian mixture contours are overlaid on the scatter plots. The populations with shorter *t_{D}* and lower Δ*I* was attributed to the GOI, and the populations having the longer *t_{D}* and higher Δ*I* was atributed to the RG. Representative, concatenated events for each sample mixture are shown. Shorter events representing the GOI are marked with asterisks. Lower panels: distribution of the capture rate for **(3C)** GOI1, and for **(3D)** GOI2. The capture rates were approximated by exponential fit to the histograms resulting in the following values: 0.61±0.01s⁻¹ and 1.17±0.05s⁻¹ for GOI1 and RG (G6PDH), respectively, 0.15±0.01s⁻¹ and 0.29±0.01s⁻¹ for GOI2 and RG, respectively. **(3E-H)** Data analysis procedure for separating populations of events based on a Gaussian mixed model (GMM). **(3E)** 2D density plots and their corresponding density histograms are generated to determine the initial conditions for the GMM (x: log-scale dwell time; y: blockage amplitude). **(3F)** The local maxima, marked by asterisks, are used as initial estimations of the means in the GMM. The covariance matrix is then calculated for both of the event parameters (blockage level and dwell time). Finally, the mixing proportions are estimated as the ratio between the maxima. **(3G)** Scatter plots represent the two populations recognized by the GMM algorithm, corresponding to the GOI2 (cyan) and the RG (G6PDH, magenta). The GMM distribution is shown as a contour plot. **(3H)** Color maps show the separation of events based on their posterior probability of belonging to the GOI or RG population.

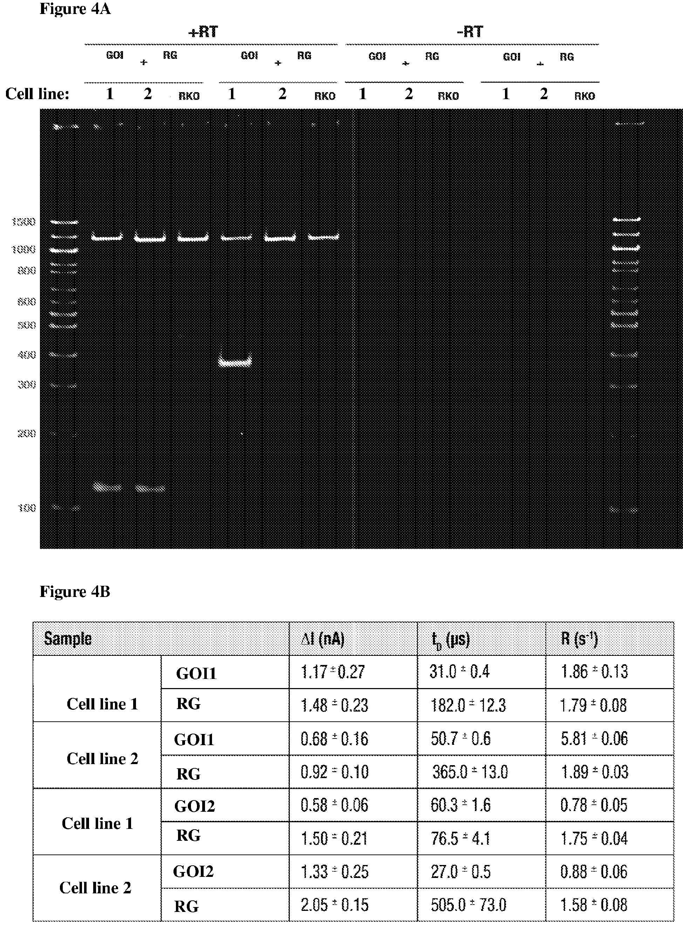

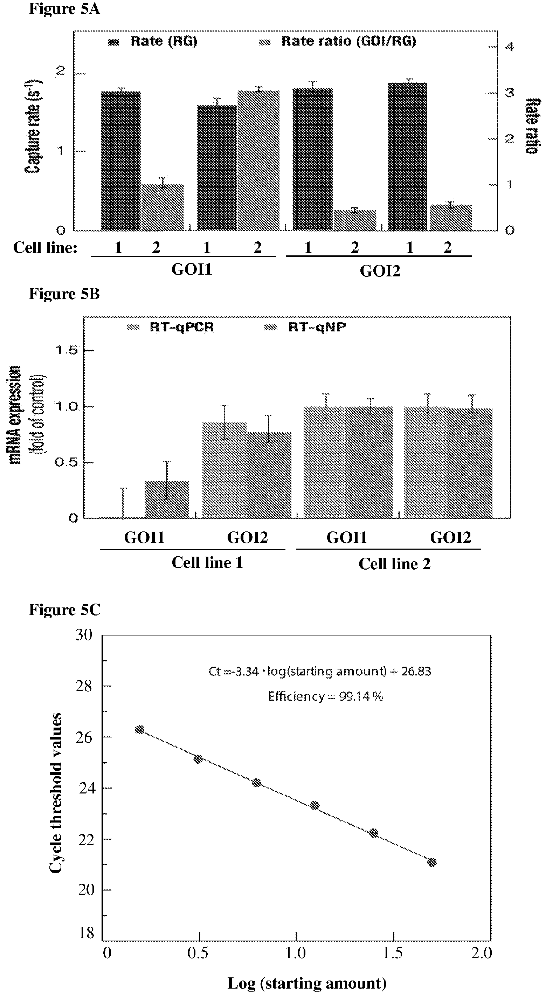

**Figures 4A****-C: (4A)** Gel electrophoresis of cDNA reverse transcripts prepared from cell lines. 50 ng DNase-treated total RNA was obtained from RKO cells or from cell line 1 or 2. The cDNA reverse transcripts were then prepared for the GOIs and the RG using the purification-free method. The samples were separated using 4% PAGE after 30 cycles of PCR amplification and imaged using the GelDoc EZ (BioRad) after SybrGold staining. **(4B)** Summary table of the nanopore results of multiplexed mixture samples prepared in **4A** and assayed in **4C.** Δ*I* denotes the event amplitude, *t_{D}* is the dwell time, and *R* is the event rate. **(4C)** NP quantification of mRNA expression levels in the cell lines. GOI1 and GOI2 and reference gene G6PDH cDNAs originated from the two cell lines, processed by RT-qNP and subjected to 16-fold amplification. Event scatter plots, representative translocation events, and histograms of the dwell times and the current blockages are shown for each of the four experiments. GMM analysis was applied to classify the events into two populations representing RG and the GOI, as indicated. Gaussian mixture contours are overlaid on top of the scatter plots. All experiments were conducted using pores with average conductance of 10.5±2 **nS.****Figures 5A****-C: (5A)** The absolute translocation event rate of G6PDH (left axis, dark grey bars), and the event rate relative to the GOI (right axis, light grey bars) measured for the four samples in **Figure 4B****.** All experiments resulted in similar event rates for the RG with an average of 1.75±0.03 s⁻¹. The relative event rate of the GOI to RG is increased from the pre- to the post- metastatic cell lines, where the most substantial increase is detected for GOI1: 0.5 s⁻¹ and 1.7 s⁻¹ for non-metastasizing and metastasizing samples, respectively. **(5B)** Comparison of the relative mRNA quantification results obtained using RT-qPCR (light grey bars) or RT-qNP (dark grey bars). The expression levels in each of the four samples were normalized to the results of the post-metastatic cell line. **(5C)** Calibration curve of GOI1 for the cell line samples, obtained by RT-qPCR. A serial dilution of starting amount of total RNA extracted from cell line 2, between 50 ng to 1.56 ng, were used to form the calibration curve.

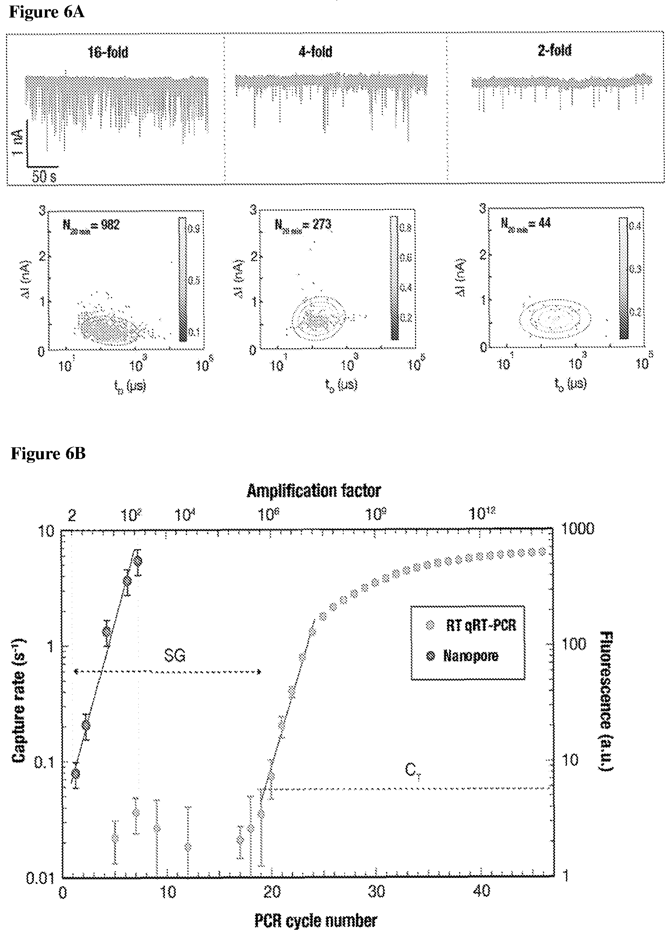

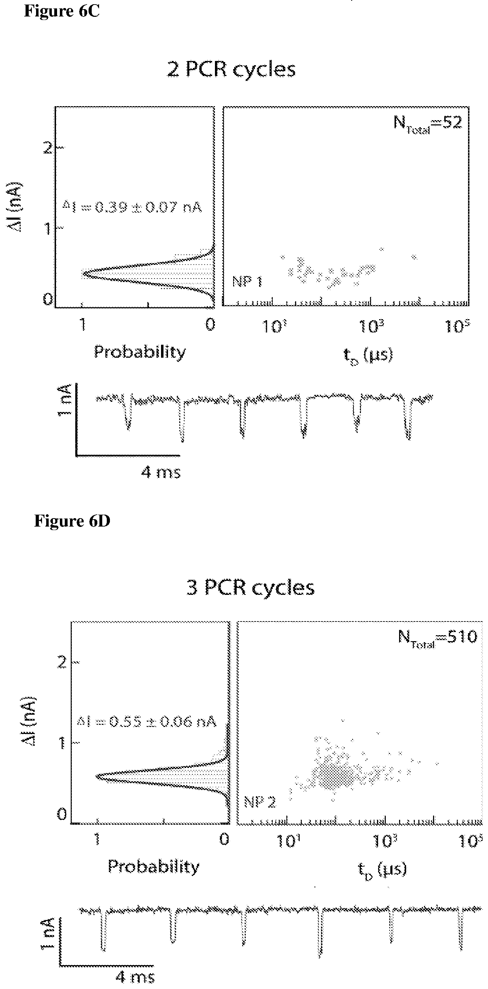

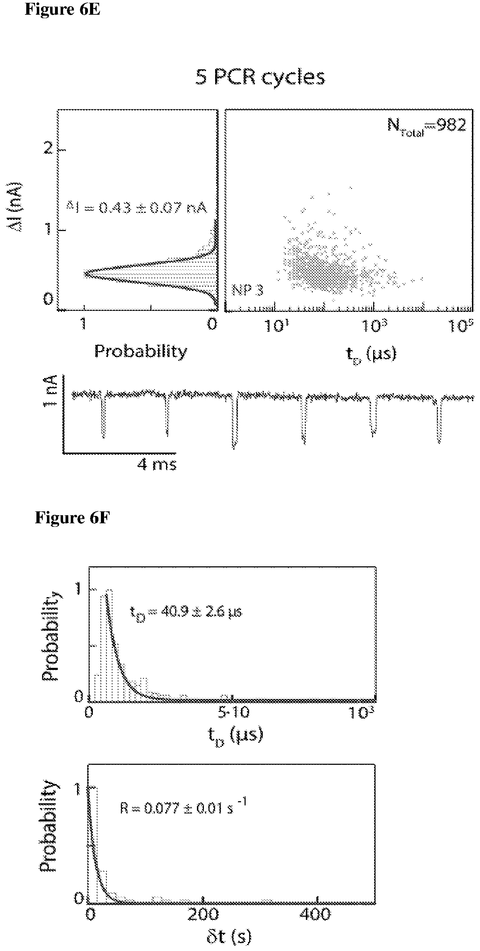

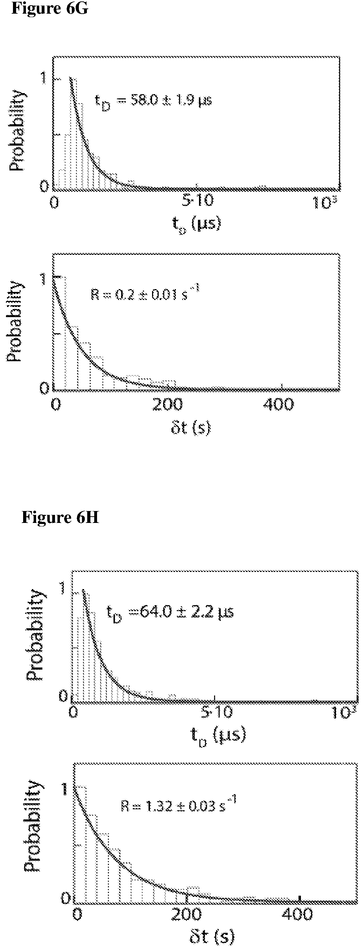

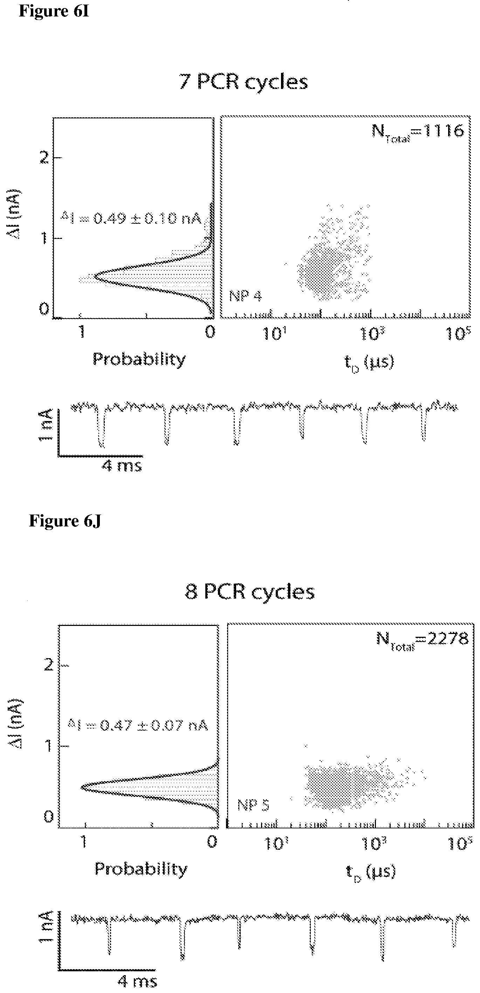

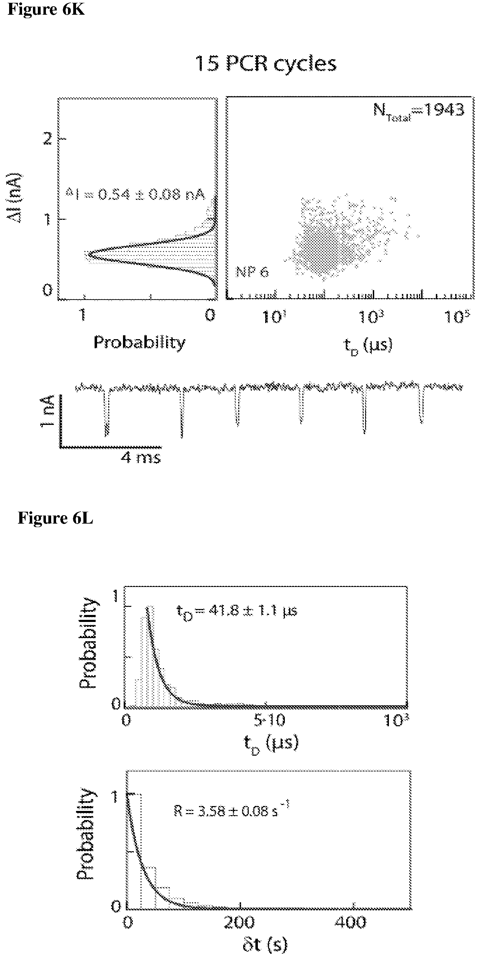

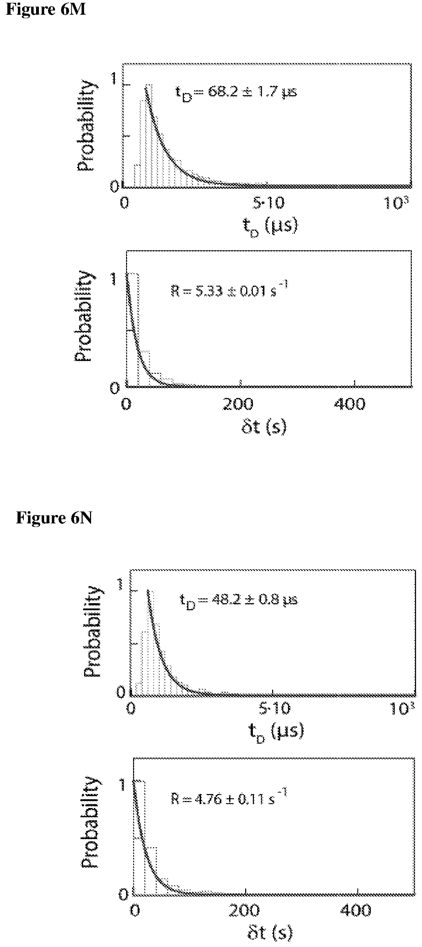

**Figures 6A****-O: (6A-B)** Evaluation of the sensitivity of RT-qNP for mRNA quantification. **(6A)** Top: raw continuous ion-current recordings of GOI1 cDNA prepared using RT-qNP, subjected to 16, 4 and 2-fold amplification and measured using 4.5 nm pores. Bottom: event diagram showing similar event amplitudes and dwell times for the three experiments, analyzed using the GMM. Dots represent the total number of events detected in the first 20 minutes of each experiment. **(6B)** Comparison of GOI expression analysis using RT-qPCR (right axis, light grey) and RT-qNP (left axis, dark grey), starting from the same source of 50 ng of total RNA extracted from cell line 2. The total fluorescence (for RT-qPCR) and the capture rate (for RT-qNP) are plotted as a function of the number of PCR cycles. The exponential regime for each method is indicated. Measurements were performed in triplicates. Error bars represent the standard deviations of measurements. The *Cₜ* value of the RT-qPCR is estimated as 20 cycles. SG indicates the sensitivity gap. **(6C-N)** RT-qNP results for GOI1 cDNA samples after optional PCR amplification. Nanopore results after PCR amplification of 2 **(6C, 6F),** 3 **(6D, 6G),** 5 **(6E, 6H),** 7 **(6I, 6L),** 8 **(6J, 6M)** and 15 **(6K, 6N)** cycles, following our purification-free RT protocol. Each sample was measured using a different nanopore. The top panel of **(6C-E)** and **(6I-K)** shows a scatter plot of the translocation events with the corresponding blockage amplitude histograms on the y-axis, as well as the concatenated current traces of representative translocation events. **(6F-H)** and (**6L-N**) show the dwell time histograms (top) and event rate histograms (bottom) from which the capture rate was calculated. Nanopore measurements were taken at 300 mV bias, and the typical conductance of pores was ~8-10 nS, corresponding to a pore diameter of ~4.5-5.2 nm). (**6O**) Amplification curves for GOI1 obtained by RT-qPCR using hybridization probes. Duplicate RT-qPCR amplification curves for GOI1 cDNA, obtained from 50 ng total RNA extracted from the cell line 2. The cycle threshold (Ct) was found to be 20.

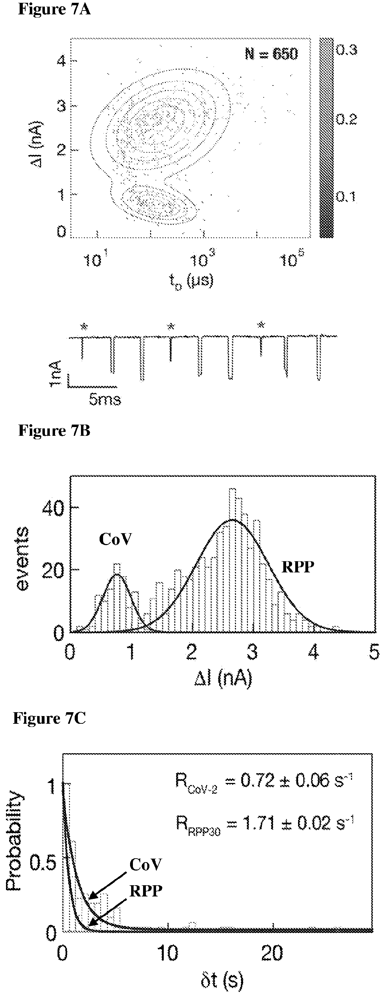

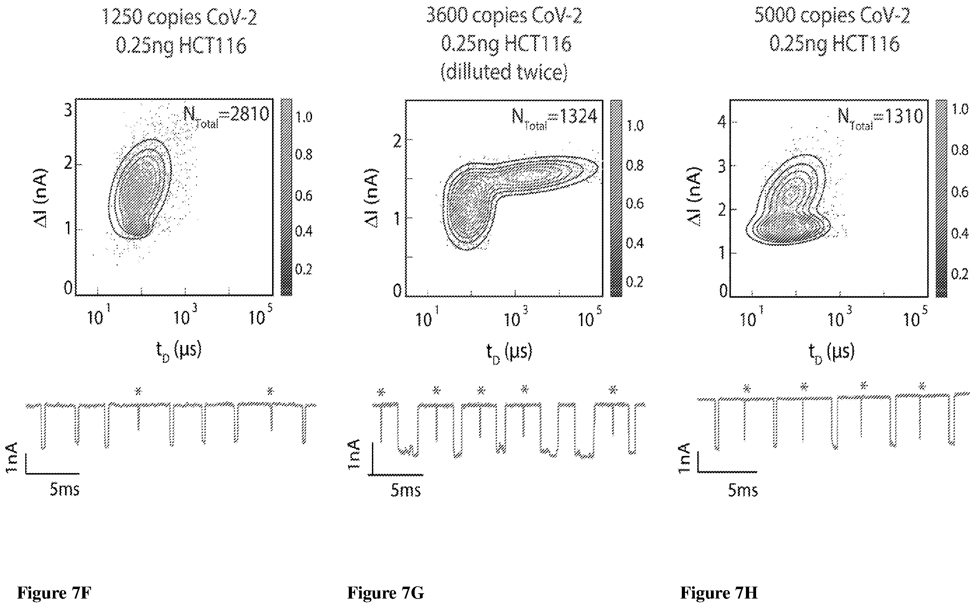

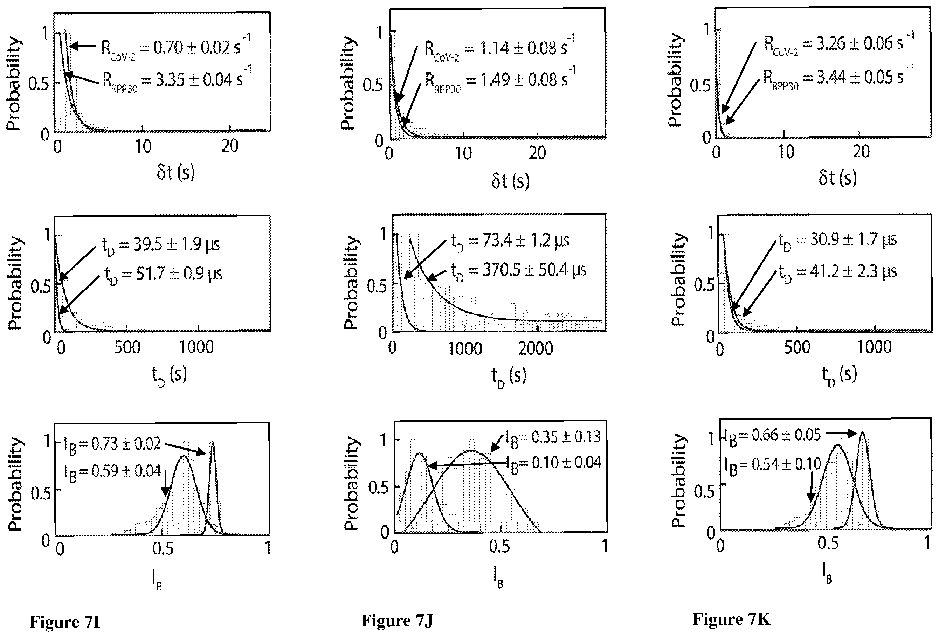

**Figures 7A****-K: (7A-D)** RT-qNP quantification of SARS-CoV-2 RNA against the human reference gene RPP30. **(7A)** Top: event diagram showing nanopore translocations of cDNA synthesized from 2500 copies SARS-CoV-2 RNA and RPP30 from 0.25 ng of total RNA from HCT116 cells. Two populations were clearly distinguished by the GMM. Bottom: concatenated ionic current traces showing representative translocation events. Short and shallow events, associated with SARS-CoV-2 cDNA, are marked with an asterisk. **(7B)** Histograms of the current blockage and **(7C)** event arrival time show two populations with distinct event rates. **(7D)** The relative event rate of SARS-CoV-2 and RPP30 detection as a function of the starting copy number of SARS-CoV-2 RNA. The amount of RPP30 was kept constant at 0.25 ng of total RNA. **(7E)** Gel electrophoresis of cDNA reverse transcripts obtained from SARS-CoV-2 RNA. cDNA fragments for each gene were prepared from 50 ng of either synthetic SARS-CoV-2 RNA or DNase-treated total RNA from HCT116 cells. The samples were processed using our purification-free method, followed by 30 cycles of PCR amplification for visualization purposes. Negative control samples (-RT) underwent the same procedure but without the RTx and Bst 2.0 enzymes. PCR negative control samples (-) contained water instead of RT template in the PCR reaction. The 'No RNA + RT' samples contained the RTx and the Bst 2.0 enzymes but contained water instead of RNA in the RT step. Hence, these can be considered as "0 copies" or "0 ng" +RT samples. The samples were separated using 4% PAGE, stained with SYBR Gold, and imaged by GelDoc EZ (BioRad). **(7F-K)** RT-qNP quantification of SARS-CoV-2 RNA against the reference gene RPP30 at three concentration ratios. The composition of each sample is shown at the top. Each sample contained the same amount of RPP30, from 0.25 ng of total RNA from HCT116 cells. **(7F-H)** Dot plots show the event diagram with representative events for **(7F)** 1250 copies, **(7G)** 3600 copies and **(7H)** 5000 copies of CoV-2. Asterisks denote the shorter translocations, which are associated with cDNA synthesized from SARS-CoV-2 RNA. (**7I-K)** Histograms of the event arrival time, dwell time and fractional current blockage for **7F-H,** respectively.

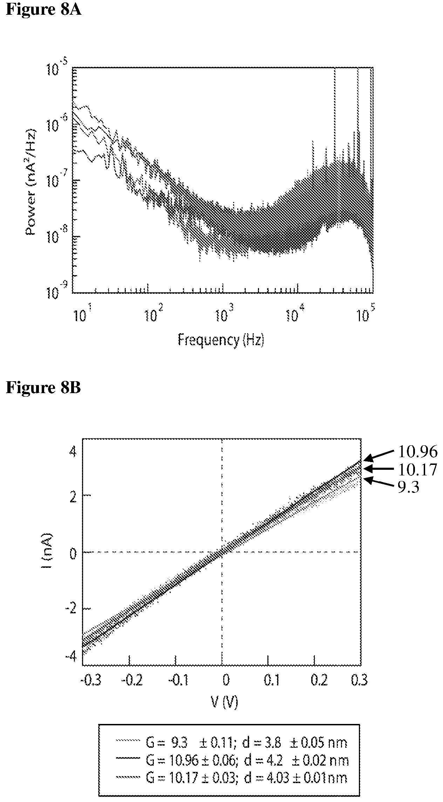

**Figures 8A****-B: Noise spectra and current-voltage (IV) curves of a nanopore. (8A)** Representative noise power spectral density (PSD) versus frequency at an applied bias of 300mV. **(8B)** Representative open pore ionic current versus voltage. A linear IV curve indicates a symmetric nanopore. All ionic currents and noise spectra were measured in a buffer containing 1M KCl and 10 mM Tris (pH 7.5).

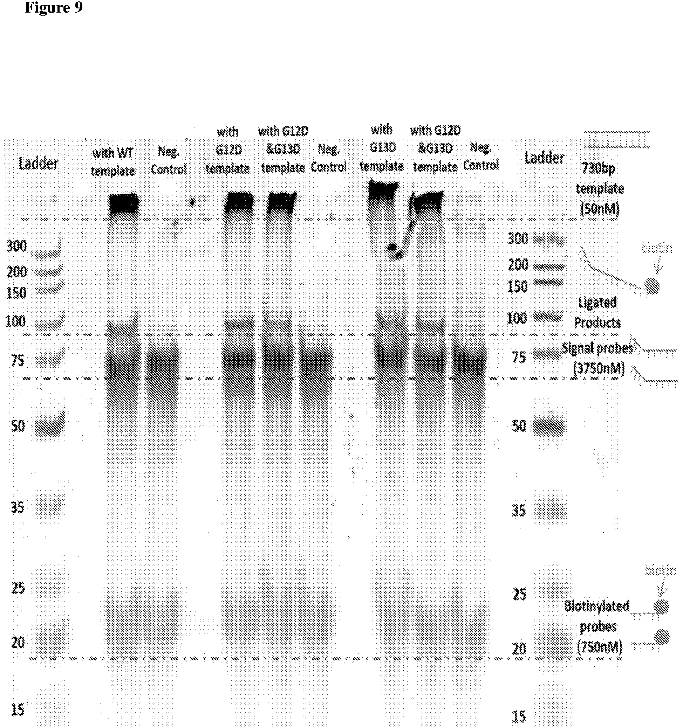

**Figure 9****:** SYBR-Gold-stained gel of template, probes and ligation products, showing the specific production of ligation product only when the proper template is mixed with the proper probes.

### DETAILED DESCRIPTION OF THE INVENTION

The present invention, in some embodiments, provides methods of quantifying a species of RNA within a sample. Methods of diagnosing a disease in a subject are also provided.

The invention is based at least in part on the surprising finding that RT-qNP (the method of the invention) offers several advantages over other common expression quantification techniques such as RT-qPCR. Firstly, by avoiding non-linear amplification, the method of the invention preserves the linear relation between the number of detected cDNA molecules and the number of RNA copies in the sample. PCR can suffer from bias and off-target amplification, which may hinder the accurate quantification of RNA at low copy number. Secondly, bypassing enzymatic amplification greatly simplifies the upstream sample treatment in RT-qNP. The compatibility with small sample volumes, short processing time and lack of thermal cycling are crucial factors for implementation of single-molecule biosensors in mobile and miniature devices.

The ability to estimate the length of each detected molecule substantially improves the signal-to-background ratio and ensures detection specificity without the need for specific labeling. This feature was used to identify cDNA from multiple genes in the same sample, enabling quantification of expression levels relative to a reference gene acting as an internal control.

Notably, the RT-qNP RNA quantification method presented is quite general and can be directly applied in many fields of biological and medical research. As an example, the method was adapted to address the acute need for high-resolution sensing of the novel SARS-CoV-2 RNA; showing that viral RNA can readily be quantified simultaneously with the human RPP30 gene used as a reference and quality control factor for the sample. Single-molecule counting of the two RNA types produces a linear relationship between the measured rate and SARS-CoV-2 RNA copy number in a range that is often encountered in clinical testing. In this case, PCR amplification was eliminated entirely, allowing direct counting of cDNA molecules. On top of the improved accuracy, the elimination of PCR amplification highly simplifies the overall biochemical assay potentially reducing the sample test time and reagents costs.

By a first aspect, there is provided a method for quantifying a first species of RNA within a sample, the method comprising:
a. receiving a sample comprising RNA;
b. contacting the sample with a first primer that hybridizes to the first species of RNA under conditions sufficient for reverse transcription (RT) of the first species of RNA to cDNA;
c. treating the sample with an RNAse;
d. contacting the sample with a second primer that hybridizes to the cDNA and producing an amplification product that is reverse complementary to the cDNA;
e. treating the sample with a proteinase comprising a net positive charge;
f. passing the amplification product through a nanopore and
g. identifying the amplification product as it passes through the nanopore;

wherein said method is devoid of a washing or isolation step after step (a),
thereby quantifying a species of RNA within a sample.

By another aspect, there is provided a method of diagnosing a disease in a subject in need thereof, the method comprising performing a method of the invention, wherein the sample is from the subject, the first RNA species is an RNA of a gene associated with the disease and detection of the first RNA species in the sample indicates the subject suffers from the disease.

By another aspect, there is provided a method of diagnosing a disease in a subject in need thereof, the method comprising:
a. receiving a sample from the subject comprising RNA;
b. contacting the sample with a first primer that hybridizes to a first species of RNA associated with the disease under conditions sufficient for reverse transcription (RT) of the first species of RNA to cDNA;
c. treating the sample with an RNAse;
d. contacting the sample with a second primer that hybridizes to the cDNA and producing an amplification product that is reverse complementary to the cDNA;
e. treating the sample with a proteinase comprising a net positive charge;
f. passing the amplification product through a nanopore and
g. identifying the amplification product as it passes through the nanopore; wherein identifying the amplification product indicates the subject suffers from the disease

wherein said method is devoid of a washing or isolation step after step (a),
thereby diagnosing a disease in a subject.

In some embodiments, the method is an in vitro method. In some embodiments, the method is a method of diagnosis. In some embodiments, the method is a method of detecting a first RNA species. In some embodiments, the quantifying is detecting. In some embodiments, the method is a method of ultrasensitive quantification. In some embodiments, the method is a method of detecting a lowly abundant RNA species.

In some embodiments, the RNA is mRNA. In some embodiments, the first RNA species is a lowly abundant RNA species in the sample. In some embodiments, the first species of RNA is RNA of a target gene. In some embodiments, the target gene is a disease-associated gene. In some embodiments, the first species of RNA is an RNA of a disease-associated gene. In some embodiments, the disease-associated gene is a disease-specific gene. In some embodiments, expression of the gene is a sample is indicative of the presence of the disease. In some embodiments, expression of the first RNA species is a sample is indicative of the presence of the disease. In some embodiments, the presence of the gene in the sample is indictive of the presence of the disease. In some embodiments, the presence of the first RNA species in the sample is indictive of the presence of the disease. In some embodiments, presence of the disease is presence of the disease in the sample. In some embodiments, presence of the disease is presence of the disease in a subject that provided the sample. In some embodiments, presence of the gene or first RNA species is presence of the gene or RNA species above a predetermined threshold.

In some embodiments, the disease is an infectious disease. In some embodiments, the first species of RNA is an RNA of an infectious agent. In some embodiments, the infectious agent is a virus. In some embodiments, the infectious agent is a bacterium. In some embodiments, the infectious agent is a parasite. In some embodiments, the infectious agent RNA is an RNA unique to the infectious agent. In some embodiments, a viral RNA is an RNA that encodes an essential viral protein. In some embodiments, the virus is SARS-CoV-2. In some embodiments, the viral RNA is an RNA of RNA-dependent RNA polymerase (RdRP). Infectious agent markers are well known in the art and any known marker may be employed. Examples of infectious agent markers include, but are not limited to, RdRP, viral spike gene/protein, viral capsid gene/protein, E gene/protein, N gene/protein, 23S rRNA, gyrB, dnaK, and recA to name but a few.

In some embodiments, the disease is a proliferative disease. In some embodiments, the proliferative disease is cancer. In some embodiments, the disease is characterized by the presence of a mutation. In some embodiments, the first species of RNA comprises the mutation. In some embodiments, the mutation is a pro-proliferative mutation. In some embodiments, the mutation is an anti-apoptotic mutation. In some embodiments, the mutation is a cancerous mutation. In some embodiments, the mutation is an oncogenic mutation. In some embodiments, disease associated gene is a cancer-associated gene. In some embodiments, cancer-associated is a cancer specific. In some embodiments, the cancer-associated gene is a cancer marker. In some embodiments, the cancer-associated mutation is a cancer inducing mutation. In some embodiments, a cancer inducing mutation is a cancer driving mutation. In some embodiments, the gene is KRAS. Examples of cancer drivers, cancer associated gene and cancer associated/causing mutations are well known in the art. Any such mutation can be screened for. Screening methods of cancer transcripts are known in the art and so long as the cancer-causing sequence is known the method of the invention can be adapted to screen for that mutation, such as is described hereinbelow. Cancer markers are well known in the art and any such marker may be employed. Examples of cancer markers include, but are not limited to KRAS, MACC1, S100A4, AFP, CA125, CA15-3, CA19-9, CEA, hCG and PSA to name but a few. Cancer driver mutations are also well known in the art and any such mutation may be investigated. Example of cancer driver mutations include, but are not limited to, KRAS G12D, KRAS G13D, BRAF D594A/E/G/HN, EGFR L858R, EGFR T790M, and P53 P72R. Cancer mutations can also be found for example in the Cancer mutations browser (intogen.org) or the Catalogue of Somatic Mutations in Cancer (COSMIC, cancer.sanger.ac.uk). In some embodiments, the mutation is in a coding region. In some embodiments, the mutation is in a transcribed region.

In some embodiments, cancer is metastatic cancer. In some embodiments the cancer is premetastatic cancer. In some embodiments, cancer is solid cancer. In some embodiments, cancer is a tumor. In some embodiments, cancer is hematopoietic cancer. In some embodiments, cancer is residual disease. In some embodiments, cancer is early detection of cancer. In some embodiments, cancer is colorectal cancer. Examples of cancer include, but are not limited to brain cancer, oral cancer, head and neck cancer, esophageal cancer, lung cancer, skin cancer, liver cancer, pancreatic cancer, bladder cancer, renal cancer, blood cancer, bladder cancer, bone cancer, breast cancer, thyroid cancer, cervical cancer, ovarian cancer, testicular cancer, retinoblastoma, gastric cancer, colorectal cancer, and uterine cancer.

In some embodiments, the sample is from a subject. In some embodiments, the subject is a subject in need of diagnosing a possible disease. In some embodiments, the subject is a mammal. In some embodiments, the subject is a human. In some embodiments, the subject is at risk for the disease. In some embodiments, the subject has been exposed to a carrier of an infectious disease. In some embodiments, the subject is a subject during a pandemic. In some embodiments, the subject is believed to have been infected by an infectious agent. In some embodiments, the subject is in need of determining if the subject has been infected by an infectious agent. In some embodiments, the subject exhibits symptoms of being infected by an infectious agent. In some embodiments, the subject has been in a region with an outbreak of infection by the infectious agent. In some embodiments, the subject has a proliferative disease and is at risk of metastasis. In some embodiments, the subject had a proliferative disease and is at risk of relapse. In some embodiments, the diagnosis is diagnosis of residual disease. In some embodiments, diagnosis is early diagnosis. In some embodiments, diagnosis is diagnosis of a proliferative disease before development of any symptoms.

In some embodiments, the sample is a solution. In some embodiments, the sample is processed into a solution. In some embodiments, the sample is a sample devoid of DNA. In some embodiments, the sample is a sample devoid of DNA polymers. In some embodiments, the sample is a sample treated with DNAse. In some embodiments, the method further comprises treating the sample with DNAse. In some embodiments, the DNAse is a DNAse enzyme. DNAses are well known in the art and any such DNAse may be used. In some embodiments, the DNAse cleaves genomic DNA. In some embodiments, the DNAse cleaves cfDNA. In some embodiments, the DNAse cleaves mitochondrial DNA. In some embodiments, the DNAse is DNAseI. In some embodiments, the sample is a sample of isolated RNA.

In some embodiments, the sample is a blood sample. In some embodiments, the sample is a bodily fluid sample. Examples of bodily fluids include, but are not limited to blood, plasma, urine, feces, cerebral spinal fluid, semen, breast milk, tumor fluid and saliva. In some embodiments, the sample is a tumor sample. In some embodiments, the sample is a liquid biopsy. In some embodiments, the sample is a biopsy. In some embodiments, the sample is tissue. In some embodiments, the sample is a tissue biopsy. In some embodiments, the saliva is spit.

In some embodiments, the sample is a swab. In some embodiments, the swab is a swab of an area of the subject. In some embodiments, the area is an area at risk for infection by the infectious agent. In some embodiments, the area is an area infected by an infectious agent. In some embodiments, the area is an area of infection. In some embodiments, the area is the inside of the nose. In some embodiments, the area is in the mouth. In some embodiments, the area is in the cheek. In some embodiments, the area is in the throat. In some embodiments, the area is a wound. In some embodiments, the area is the eye. In some embodiments, the area is the anus. In some embodiments, the area is the urethra. In some embodiments, the area is the vagina. In some embodiments, the area is an ear.

In some embodiments, receiving comprising receiving a swab. In some embodiments, receiving comprising receiving a solution generated from a swab. In some embodiments, the swab is generated from swabbing an area of a subject. In some embodiments, the solution is a transfer solution in which the swab is dipped or incubated. In some embodiments, transfer solution is TE. In some embodiments, transfer solution is LB. In some embodiments, transfer solution comprises nutrients sufficient for culture of cells. In some embodiments, the culture is liquid culture. In some embodiments, the cells are bacterial cells. Transfer solution for swabs is well known in the art, and any such transfer solution may be used. In some embodiments, the sample is the solution.

In some embodiments, receiving comprises lysing cells in the solution. In some embodiments, the solution is the transfer buffer after incubation with the swab. In some embodiments, receiving comprises adding lysis solution to the transfer solution. In some embodiments, the lysing solution lyses cells. In some embodiments, the lysing solution comprises 3M guanidine. In some embodiments, the lysing solution is a hypotonic solution. In some embodiments, the lysing solution is a hypertonic solution. In some embodiments, the lysis buffer is RNA extraction lysis buffer. Lysing solutions are well known in the art, and any such lysis solution may be used.

Methods for swab collection and transfer and lysis can be found, for example in "Swab Sample Transfer for Point-Of-Care Diagnostics: Characterization of Swab Types and Manual Agitation Methods", Panpradist et al., 2014, PLoS one; 9(9): e105786; CDC guidelines for Clinical Specimens (cdc.gov/coronavirus/2019-ncov/lab/guidelines-clinical-specimens.html) and WHO interim guidance 19 March 2020: Laboratory testing for coronavirus disease (COVID-19) in suspected human cases (apps.who.int/iris/rest/bitstreams/1271387/retrieve).

In some embodiments, the sample comprises cells. In some embodiments, the cells are from the subject. In some embodiments, the cells are from the swab. In some embodiments, the cells are lysed. In some embodiments, the method comprises lysing the cells. In some embodiments, the sample is a cellular lysate. In some embodiments, the method comprises contacting the cell lysate with a DNAse. In some embodiments, the receiving comprises receiving a cellular lysate and contacting the lysate with DNAse to produce a sample comprising RNA and substantially devoid of DNA.

In some embodiments, substantially devoid comprises less than 1, 0.5, 0.1, 0.01, 0.001 or 0.0001% DNA in the total of nucleic acid molecules. Each possibility represents a separate embodiment of the invention. It will be understood by a skilled artisan that a DNAse will cleave DNA polymers down to individual DNA nucleotides or dinucleotides. This is to be understood as substantially devoid of DNA. In some embodiments, substantially devoid of DNA is devoid of DNA polymers. In some embodiments, a nucleic acid polymer is a nucleic acid chain comprising at least 3 nucleotides. In some embodiments, a nucleic acid polymer is a nucleic acid chain comprising at least 2 nucleotides. In some embodiments, a nucleic acid molecule is a DNA or RNA molecule. In some embodiments, a molecule is a polymer.

In some embodiments, the primer is a DNA primer. In some embodiments, the primer is an RNA primer. In some embodiments, the first primer hybridizes to the first RNA species. In some embodiments, the first primer specifically hybridizes to the first RNA species. In some embodiments, the primer is a forward primer. In some embodiments, the primer is reverse complementary to an RNA. In some embodiments, the primer can hybridize to an RNA. In some embodiments, the primer comprises a region reverse complementary to an RNA. In some embodiments, the region of reverse complementarity is the 3' end of the primer. In some embodiments, the primer comprises a length of at least 10, 12, 14, 15, 16, 18, 20, 22, 24, 25, 26, 28, or 30 nucleotides. Each possibility represents a separate embodiment of the invention. In some embodiments, the primer comprises a length of at most, 20, 22, 24, 25, 26, 28, 30, 32, 34, 35, 36, 38, 40, 42, 44, 45, 46, 48, or 50 nucleotides. Each possibility represents a separate embodiment of the invention.

In some embodiments, the first primer is specific to the first RNA species. In some embodiments, specific is specific hybridization. In some embodiments, hybridization is perfect complementarity. In some embodiments, the first primer hybridizes to the first RNA species with at least 100, 99, 97, 95, 90, 85, or 80% complementarity. Each possibility represents a separate embodiment of the invention. In some embodiments, the first primer hybridizes to the first RNA species with 100% complementarity. In some embodiments, the first primer is specific to the first RNA species and to no other RNAs. In some embodiments, no other RNAs is no other RNAs in the sample. In some embodiments, no other RNAs is no other RNAs in a cell of the subject. In some embodiments, no other RNAs is no other RNAs in the infectious agent.

In some embodiments, the contacting is under conditions sufficient for reverse transcription (RT). In some embodiments, the contacting is incubating. In some embodiments, the RT is RT of the first RNA species to cDNA. In some embodiments, the RT is RT of the first RNA species to a first strand of cDNA. In some embodiments, the RT produces a first cDNA strand. In some embodiments, the first cDNA strand is complementary to the first species of RNA. In some embodiments, the first cDNA strand is complementary to at least a portion of the first RNA species. In some embodiments, the first cDNA strand comprises the first primer. In some embodiments, the first primer is the 5' terminus of the first cDNA strand. In some embodiments, step (b) comprises contacting the sample with a reverse transcriptase. In some embodiments, step (b) comprises contacting the sample with a polymerase. In some embodiments, the polymerase is DNA polymerase. In some embodiments, the reverse transcriptase is warmstart RTx. In some embodiments, step (b) comprises contacting the sample with free DNA nucleotides.

In some embodiments, incubation is incubation under suitable conditions. In some embodiments, contacting is contacting under suitable conditions. In some embodiments, the incubating is under conditions suitable for binding of RNA from the sample to the primer. In some embodiments, the incubating is under conditions suitable for hybridization of RNA from the sample to the primer. In some embodiments, the DNAse is inactivated prior to addition of the primer. In some embodiments, the inactivation is heat inactivation. In some embodiments, the hybridization is RNA to DNA hybridization. In some embodiments, suitable conditions are conditions suitable for primer hybridization to an RNA. In some embodiments, suitable conditions are conditions suitable for RNA to DNA hybridization.

RT and RT-PCR are well known in the art. Exemplary primers are provided in Tables 1-3. Methods of designing primers for amplifying specific sequences are well known, and programs, such as Primer3 for a non-limiting example, can be employed. In some embodiments, the RT-PCR is a single round of RT-PCR. In some embodiments, the RT-PCR produces a strand of cDNA reverse complementary to an RNA. In some embodiments, the RT-PCR is done with the forward primer. In some embodiments, reverse-transcription is RT-PCR. In some embodiments, reverse-transcription comprises adding a polymerase. In some embodiments, the polymerase is Taq polymerase. Any polymerase with 5' to 3' polymerase activity may be used. In some embodiments, reverse-transcription comprises adding free nucleotide bases. All 4 bases, A, T, C and G are added to allow polymerization. In some embodiments, a PCR buffer is added. Kits and reagents for reverse-transcription are well known in the art an any such kits may be used, for example first strand synthesis kits can be used. Such kits often comprise a random binding agent, such as random hexamers or random primers; it will be understood that the first primer is to be used instead. This produces only a first cDNA strand that is the reverse complement of the first species of RNA and no other cDNAs. In some embodiments, the reverse-transcription reaction is a first strand synthesis reaction. Conditions for reverse-transcription including heating and cooling steps are well known. Standard reverse-transcription and/or first strand synthesis may be used. In some embodiments, the reverse-transcription produces a cDNA strand thereby preparing cDNA from a sample comprising RNA. In some embodiments, RT is a single round of RT. In some embodiments, RT produces about a 1:1 ratio of first RNA species molecules to first cDNA strand molecules.

In some embodiments, the method comprises contacting the sample with an RNAse. In some embodiments, the RNAse is an RNAse enzyme. In some embodiments, contacting the sample is treating the sample with an RNAse. In some embodiments, contacting the sample is incubating the sample with an RNAse. In some embodiments, the RNAse produces a sample substantially devoid of RNA. In some embodiments, the RNAse produces a sample comprising the first cDNA strand and substantially devoid of RNA. In some embodiments, devoid of RNA is devoid of RNA polymers. It will be understood that an RNAse will cleave RNA polymers into RNA nucleotides or dinucleotides. In some embodiments, substantially devoid is devoid. In some embodiments, substantially devoid is devoid of polymers but comprising single nucleotides. In some embodiments, substantially devoid is devoid of polymers but comprising single nucleotides or dinucleotides.

In some embodiments, step (c) occurs after step (b). In some embodiments, step (c) occurs before step (d). In some embodiments, step (c) occurs after step (d). In some embodiments, the RNAse treatment produces a sample comprising the first cDNA strand and the amplification product. In some embodiments, the RNAse treatment produces a sample comprising the first cDNA strand and the amplification product and substantially devoid of RNA. In some embodiments, step (b) and step (d) are a single step. In some embodiments, the reverse transcriptase is a DNA polymerase. In some embodiments, the first primer and second primer are added together.

In some embodiments, step (d) comprises contacting the sample with a second primer. In some embodiments, the second primer hybridizes to the first cDNA strand. In some embodiments, the second primer is specific to the first cDNA strand. In some embodiments, the second primer is reverse complementary to the first cDNA strand. In some embodiments, the second primer is homologous to a region of the first RNA species. In some embodiments, the first and second primers are a primer pair. In some embodiments, the first and second primers produce an amplicon of a given length. In some embodiments, the amplicon is the amplification product.

In some embodiments, the second primer hybridizes to the first cDNA strand with at least 100, 99, 97, 95, 90, 85, or 80% complementarity. Each possibility represents a separate embodiment of the invention. In some embodiments, the second primer hybridizes to the first cDNA strand with 100% complementarity. In some embodiments, the second primer is specific to the first cDNA strand and to no other DNAs. In some embodiments, the second primer is specific to the first cDNA strand and to no other DNAs or RNAs. In some embodiments, no other DNAs is no other cDNAs. In some embodiments, no other DNAs is no other DNAs in the sample. In some embodiments, no other DNAs is no other DNAs in a cell of the subject. In some embodiments, no other DNAs is no other DNAs in the infectious agent.

In some embodiments, producing an amplification product comprises performing amplification to produce the amplification product. In some embodiments, the amplification is PCR amplification. In some embodiments, the amplification is a single round of amplification. In some embodiments, the amplification produces about a 1:1 ratio of first cDNA strands to amplification product. In some embodiments, the amplification produces on average a single amplification product for each first cDNA strand. In some embodiments, the amplification produces on average a single amplification product for each molecule of the first RNA species. In some embodiments, the amplification is a single cycle of amplification. In some embodiments, the amplification is 1-2 rounds of amplification. In some embodiments, the amplification is 1-3 rounds of amplification. In some embodiments, the amplification is 1-4 rounds of amplification. In some embodiments, the amplification is 1-5 rounds of amplification. In some embodiments, the amplification is not more than 5 rounds of amplification.

In some embodiments, the amplification product is derived from the cDNA. In some embodiments, the amplification product is derived from the RNA. In some embodiments, the amplification product is derived from the first RNA species. In some embodiments, the amplification product comprises a first termini that is identical to a sequence of the first primer and a second termini that is a reverse complement to a sequence of the second primer. In some embodiments, a reverse complement is reverse complementary. In some embodiments, the amplification product comprises a first termini that is a reverse complement to a sequence of the first primer and a second termini that is identical to a sequence of the second primer. It will be understood that the amplification product is a PCR product produced the by first and second primers and will have a given length defined by the distance from the end of the first primer to the end of the second primer.

In some embodiments, the amplification product is shorter than the first RNA species. In some embodiments, shorter is shorter by a length sufficient to allow the first RNA species to be distinguished from the amplification product by the difference in dwell time through a nanopore. A skilled artisan will appreciate that a nanopore sensor can measure the dwell time of molecules within the nanopore, and that nucleic acid molecules dwell time will be proportional to their length. Thus, longer nucleic acids will have a longer dwell time and shorter nucleic acids a shorter dwell time. Further, the difference in length needed to distinguish between two molecules will depend on the sensitivity of the nanopore sensor and the conditions at which the molecules are passed through the nanopore. So, a skilled artisan would choose a length difference that is reliably detectable. In some embodiments, shorter is at least 5, 10, 15, 20, 25, 30, 35, 40, 45, 50, 60, 70, 80, 90, 100, 200, 250, 300, 400, 500, 600, 700, 750, 800, 900 or 1000 nucleotides shorter. Each possibility represents a separate embodiment of the invention. In some embodiments, shorter is at least 20 nucleotides shorter. In some embodiments, shorter is at least 30 nucleotides shorter. In some embodiments, shorter is at least 40 nucleotides shorter. In some embodiments, shorter is at least 45 nucleotides shorter.

In some embodiments, the amplification product is at most 500, 1000, 2000, 3000, 4000, 5000, 6000, 7000, 8000, 9000, or 10000 nucleotides. Each possibility represents a separate embodiment of the invention. In some embodiments, the amplification product is at most 500 nucleotides. In some embodiments, the amplification product is at most 1000 nucleotides. In some embodiments, the amplification product is at least 50, 100, 150, 200, 250, 300 or 350 nucleotides. Each possibility represents a separate embodiment of the invention. In some embodiments, the amplification product is at least 100 nucleotides. In some embodiments, the amplification product is at least 200 nucleotides. In some embodiments, the amplification product is at least 300 nucleotides. In some embodiments, the amplification product is the first amplification product. In some embodiments, the amplification product is the second amplification product.

In some embodiments, the amplification product does not comprise a detectable moiety. In some embodiments, the amplification does not comprise integrating a detectable moiety into the amplification product. In some embodiments, the amplification product is not labeled. In some embodiments, the amplification product is naked DNA. In some embodiments, the amplification product is detectable as it passes through the nanopore only do to its blocking of the nanopore. In some embodiments, blocking is blocking current through the nanopore. In some embodiments, blocking is blocking electrical charge. In some embodiments, the amplification product is not fluorescent.

In some embodiments, the method further comprises treating the sample with a proteinase. In some embodiments, a proteinase is a proteinase enzyme. In some embodiments, the proteinase is a protease. In some embodiments, the proteinase is a non-specific proteinase. In some embodiments, the proteinase cleaves proteins into amino acids. In some embodiments, the proteinase is resistant to autolysis. In some embodiments, the proteinase autolyzes. In some embodiments, the treatment with the proteinase produces a sample substantially devoid of polypeptides. In some embodiments, the treatment with the proteinase produces a sample substantially devoid of polypeptides other than the proteinase. In some embodiments, treating with a proteinase is under condition sufficient to allow proteinase activity. In some embodiments, proteinase activity comprises degradation of polypeptides to single amino acids. In some embodiments, the conditions are conditions sufficient for protection of the proteinase from autolysis. In some embodiments, proteinase is not active to autolyze.

In some embodiments, the proteinase comprises a net positive charge. In some embodiments, the proteinase is positively charged. In some embodiments, the proteinase is proteinase K. Proteinases are well known in the art and a skilled artisan can select one with the proper characteristics (e.g. charge and cleavage). In some embodiments, an enzyme carrying a net positive charge is an enzyme that when in solution through which electrical current is passed migrates toward a negative pole. Thus, it will be understood that the proteinase when applied to a nanopore apparatus will not migrate to the nanopore, but rather will be attracted to the negative pole in the first reservoir.

In some embodiments, the method is devoid of a washing step. In some embodiments, the method is devoid of an isolation step. In some embodiments, the method does not comprise a washing step. In some embodiments, the method does not comprise an isolation step. In some embodiments, the method after step (a) is devoid of or does not comprises an isolation step. Washing and isolation steps can lead to loss of product or starting material and greatly reduce the accuracy and detection threshold. Thus, not having these steps allows for a highly accuracy and ultrasensitive method of detection.

In some embodiments, the method comprises a dissociation step. In some embodiments, the dissociation comprises heating. In some the first cDNA strand is dissociated from the RNA species. In some embodiments, the amplification product is dissociated from the first cDNA strand. In some embodiments, one strand of the amplification product is dissociated from a second strand of the amplification product. In some embodiments, dissociation occurs before RNAse treatment. In some embodiments, the method is devoid of a dissociation step.

In some embodiments, the method comprises passing the amplification product through a nanopore. In some embodiments, the nanopore is part of a nanopore apparatus. In some embodiments, the nanopore is a solid state nanopore. In some embodiments, the nanopore is a plasmonic nanopore. In some embodiments, the nanopore is an ion-conducting nanopore. In some embodiments, the nanopore is a plasmonic nanowell. In some embodiments, the amplification product is double stranded DNA. In some embodiments, the amplification product is single stranded DNA.

The exact size of the nanopore is not essential to the performance of the method. It is sufficient that the nanopore sensor can detect the dwell time of the amplification products. In some embodiments, the nanopore comprises a diameter not greater than 1, 2, 3, 4, 5, 7, 10, 15, 20, 15, 30, 35, 40, 45, 50, 55, 60, 65, 70, 75, 80, 85, 90, 95, 100, 110, 120, 130, 140, 150 nm. Each possibility represents a separate embodiment of the invention. In some embodiments, the nanopore comprises a diameter not greater than 5 nm. In some embodiments, the nanopore comprises a diameter not greater than 7 nm. In some embodiments, the nanopore comprises a diameter not greater than 20 nm. In some embodiments, the nanopore comprises a diameter not greater than 100 nm. In some embodiments, the nanopore comprises a diameter of about 3 nm. In some embodiments, the nanopore comprises a diameter of about 4 nm. In some embodiments, the nanopore comprises a diameter of about 5 nm. In some embodiments, the nanopore comprises a diameter between 0.5 and 3, 0.5 and 4, 0.5 and 5, 0.5 and 7, 0.5 and 10, 0.5 and 15, 0.5 and 20, 1 and 3, 1 and 4, 1 and 5, 1 and 7, 1 and 10, 1 and 15, 1 and 20, 3 and 4, 3 and 5, 3 and 7, 3 and 10, 3 and 15, 3 and 20, 4 and 5, 4 and 7, 4 and 10, 4 and 15, 4 and 20, 5 and 7, 5 and 10, 5 and 15, or 5 and 20 nm. Each possibility represents a separate embodiment of the invention. In some embodiments, the nanopore comprises a diameter between 3 and 4 nm. The width of a nucleic acid molecule is ~1 nm therefore nanopores above this size are generally ideal. In some embodiments, nanopores of greater than 20 nm do not provide sufficient resolution to distinguish between amplicons of different sizes.

In some embodiments, the nanopore is part of a nanopore apparatus. In some embodiments, the nanopore is in a film. The production of nanopores in a film is well known in the art. Fabrication of nanopores in thin membranes has been shown in, for example, Kim et al., Adv. Mater. 2006, 18 (23), 3149 and Wanunu, M. et al., Nature Nanotechnology 2010, 5 (11), 807-814. Further, methods of such fabrication of films in silicon wafers, and methods of producing nanopores therein are provided herein in the Materials and Methods section. In some embodiments, the nanopore is produced with a transition electron microscope (TEM). In some embodiments, the nanopore is produced with a high-resolution aberration-corrected TEM or a noncorrected TEM. In some embodiments, the film is a membrane.

According to some embodiments, the nanopore apparatus comprises a film, and wherein the film comprises at least one nanopore. In some embodiments, the nanopore apparatus further comprises a first and a second fluidic reservoir separate by the film and connected via the nanopore. In some embodiments, the nanopore apparatus further comprises first and second electrodes configured to electrically contact fluid placed in the first reservoir and fluid placed in the second reservoir, respectively. In some embodiments, the electrodes are configured to generate an electrical current that drives an amplification product to be analyzed through the nanopore. In some embodiments, the negative electrode is placed in the first reservoir. It will be understood by a skilled artisan that negatively charged DNA will flow away from the negative electrode in the first reservoir, through the nanopore and to the positive electrode in the second reservoir. In some embodiments, the electrode in the second reservoir is a positive electrode. In some embodiments, an electrode is a pole. In some embodiments, the positively charged proteinase will stay in the first reservoir and will not pass through the nanopore.

In some embodiments, passing the amplification product comprises depositing the sample into the first reservoir of the nanopore apparatus. In some embodiments, the first reservoir comprises an ionic solution. In some embodiments, the first reservoir comprises a solution suitable for transfer through the nanopore. In some embodiments, the sample is a solution and the solution is mixed into the solution in the first reservoir. In some embodiments, the passing is by running electrical current from the first reservoir to the second reservoir via the nanopore. In some embodiments, the electoral current is run from a first electrode to a second electrode. In some embodiments, the first electrode is in the first reservoir and the second electrode is in the second reservoir.

In some embodiments, the method further comprises identifying the amplification product as it passes through the nanopore. In some embodiments, the amplification product is identified by its dwell time. It will be understood by a skilled artisan that since the dwell time is proportional to length of the molecule, the amplification product can be identified by its specific dwell time. Single nucleotides and/or amino acids will not produce a significant signal and thus will not appear as the amplification product. Similarly, two products can be distinguished by their dwell time. Thus, if the first cDNA strand were to translocate through the nanopore it could be distinguished due to its longer dwell time.

In some embodiments, the nanopore is naked in that it does not comprise a protein for facilitating transfer through the nanopore. In some embodiments, the amplification product passes through the nanopore via the electrical current generated by the electrodes. In some embodiments, the amplification product is denatured. In some embodiments, the amplification product is not denatured. In some embodiments, the nanopore apparatus further comprises a sensor or detector for detecting dwell time of molecules as they pass through the nanopore. In some embodiments, dwell time of the amplification products is measured. In some embodiments, measuring dwell time is measuring change in electrical current through the nanopore. In some embodiments, measuring dwell time is measuring change in electrical current at the nanopore. In some embodiments, identification by dwell time comprises measuring a change in electrical current through the nanopore. In some embodiments, identification by dwell time comprises measuring a change in electrical current at the nanopore. In some embodiments, sensor or detector is a current sensor or detector. In some embodiments, identification by dwell time is measuring dwell time. In some embodiments, measuring dwell time is measuring the duration of a change in electrical current. In some embodiments, the change is at the nanopore. In some embodiments, the change is through the nanopore. In some embodiments, the duration of the change is proportional to the length of the molecule passing through the nanopore. In some embodiments, the duration of the change is proportional to the length of the amplification product. In some embodiments, a longer duration is indicative of a longer molecule. In some embodiments, a shorter duration is indicative of a shorter molecule. In some embodiments, the amplification product is identified by its length.

In some embodiments, identifying the amplification product comprises differentiating the amplification product from a free DNA nucleotide. In some embodiments, identifying the amplification product comprises differentiating the amplification product from a free RNA nucleotide. In some embodiments, identifying the amplification product comprises differentiating the amplification product from a free amino acid. In some embodiments, identifying the amplification product comprises differentiating the amplification product from a first cDNA strand. In some embodiments, identifying the amplification product comprises differentiating the amplification product from the first RNA species. In some embodiments, identifying the amplification product comprises differentiating the amplification product from a off target product. If the primers were to erroneously amplify an off target, this target could still be distinguished due to its different length from the actual target amplicon. In some embodiments, identifying the amplification product comprises differentiating the amplification product from a first RNA species from an amplification product from a second RNA species.

In some embodiments, the method of the invention can also be used to simultaneously identify the presence of a second RNA species within the sample. In some embodiments, the second RNA species is a control species. In some embodiments, the second RNA is a second disease-associated RNA. By detecting the control species there is an internal control for the proper completion of all of the RT and amplification steps. Thus, even in a sample negative for the first RNA species, the control informs that the whole process is functioning and that the negative is a true negative and not a failure of the method. In some embodiments, the second RNA species is RNA of a control gene. In some embodiments, the control gene is a gene of the subject. In some embodiments, the control gene is a human gene. In some embodiments, the control gene is a gene of a healthy cell. In some embodiments, the control RNA is an RNA produced in a non-diseased cell. In some embodiments, the control RNA is an RNA produced by a non-diseased cell. In some embodiments, the control gene is a housekeeping gene. In some embodiments, the control gene is glucose-6-phosphate dehydrogenase (G6PDH). In some embodiments, the control gene is Ribonuclease P/MRP subunit P30 (RPP30). Control and housekeeping genes are well known in the art and any such gene may be used.

In some embodiments, the method further comprises contacting the sample with a third primer. In some embodiments, step (b) comprises contacting the sample with a third primer. In some embodiments, the third primer hybridizes to a second species of RNA. In some embodiments, the second primer produces a first cDNA strand complementary to the second RNA species. In some embodiments, the second primer produces a first cDNA strand complementary to at least a portion of the second RNA species. In some embodiments, the second primer produces a second first cDNA strand. In some embodiments, the first primer produces a first first cDNA strand. In some embodiments, the first cDNA strand complementary to the second RNA species comprises the second primer.

In some embodiments, the method further comprises contacting the sample with a fourth primer. In some embodiments, the method further comprises in step (d) contacting the sample with a fourth primer. In some embodiments, the fourth primer hybridizes to the second first cDNA strand. In some embodiments, the fourth primer hybridizes to the first cDNA strand complementary to the second species of RNA. In some embodiments, the fourth primer produces an amplification product. In some embodiments, the fourth primer and third primer produce an amplification product. In some embodiments, the amplification product is a second amplification product. In some embodiments, the first primer and second primer produce a first amplification product.

In some embodiments, the second amplification product comprises a first termini that is identical to a sequence of the third primer and a second termini that is a reverse complement of a sequence of the fourth primer. In some embodiments, the second amplification product comprises a first termini that is a reverse complement to a sequence of the third primer and a second termini that is identical to a sequence of the fourth primer. In some embodiments, the second amplification product is derived from the second species of RNA. In some embodiments, second amplification product is derived from the second first cDNA strand. In some embodiments, the first amplification product and the second amplification produce differ in length by at least 20 nucleotides. In some embodiments, the first amplification product and the second amplification produce differ in length by at least 100 nucleotides. In some embodiments, the first amplification product and the second amplification product differ in length by an amount sufficient to allow the first amplification product to be distinguished from the second amplification product by the difference in dwell time through a nanopore. In some embodiments, a sufficient length is at least 20 nucleotides. In some embodiments, a sufficient length is at least 100 nucleotides. In some embodiments, a sufficient length is at least 5, 10, 15, 20, 25, 30, 35, 40, 45, 50, 60, 70, 80, 90, 100, 200, 250, 300, 400, 500, 600, 700, 750, 800, 900 or 1000 nucleotides. Each possibility represents a separate embodiment of the invention.

The second RNA species can be distinguished from the first RNA species by designing primers that produce amplicons with different lengths. The amplification products are thus distinguishable by their different dwell times through the nanopore. Designing amplicons of a desired length is well known in the art and indeed many primer-design programs (e.g., Primer3) allow the setting of the amplicon length. Thus, a skilled artisan can easily design primers for the first RNA species and the second RNA species that produce amplification products of a different length that can be easily distinguished as they pass through the nanopore. Indeed, even larger numbers of RNA species (and thus genes) can be simultaneously analyzed so long as the amplicon size (amplification product size) is sufficiently different as to be distinguishable by dwell time.

In some embodiments, the method comprises quantifying at least two RNA species in the sample. In some embodiments, the method comprises quantifying at least three RNA species in the sample. In some embodiments, the method comprises quantifying at least 2, 3, 4, 5, 6, 7, 8, 9 or 10 different RNA species in the sample. In some embodiments, a single RNA species is quantified using two different sets of primers that hybridizes in different regions of the RNA molecule and produce different amplification products that can be distinguished by dwell time in the nanopore. Regardless of the number of species quantified each different amplification product produced is to be of a sufficiently different length so as to distinguish each amplification product from the other amplification products.

In some embodiments, identification of the amplification product is indicative of the presence of the RNA species within the sample. In some embodiments, each instance of an amplification product passing through the nanopore is counted and the amount of the amplification product in the sample is quantified. In some embodiments, the quantity of amplification product is proportional to the quantity of RNA species. In some embodiments, the quantity of amplification product is equal to the quantity of the RNA species. In some embodiments, the presence of a single amplification product is indicative of the presence of the RNA species. In some embodiments, expression or presence of the RNA species is indicative of the presence of a disease. In some embodiments, expression or presence of the gene is indicative of the presence of a disease. In some embodiments, expression of a gene or RNA species above a predetermined threshold indicates the presence of a disease. In some embodiments, the expression or presence is in the sample. In some embodiments, detection beyond a predetermined threshold indicates the presence of a disease. In some embodiments, the predetermined threshold is zero expression.

In some embodiments, the first species of RNA comprises a mutation. In some embodiments, the mutation is a point mutation. In some embodiments, the mutation is an insertion. In some embodiments, the mutation is a deletion.

In some embodiments, the method further comprises contacting the amplification products with a first probe. In some embodiments, the first probe is complementary to the amplification product. In some embodiments, complementary is perfectly complementary. In some embodiments, the first probe is perfectly complementary to an amplification product comprising the mutation or comprising a nucleotide reverse complementary to the mutation. It will be understood by a skilled artisan that the amplification products maybe identical in sequence to the RNA (thought they are DNA and thus will have thymidine in place of uracil) or may be reverse complementary to the RNA. When reference is made to the mutation within the RNA being present in the amplification products it will be understood that this also refers to an amplification product that is reverse complementary to the RNA and comprises the reverse complement of the mutation. In some embodiments, the first probe is perfectly complementary except for the base at the position of the mutation to an amplification product that does not comprise the mutation. In some embodiments, the first probe comprises a terminal nucleotide that is complementary to the mutation. In some embodiments, the position in the first probe that is complementary to the mutation is a terminal position. In some embodiments, terminal is 3' terminal. In some embodiments, terminal is 5' terminal. In some embodiments, terminal is 5' terminal. Thus, the first probe starts or ends with a base that is the same as/complementary to the mutation. In some embodiments, the mutation is a deletion and the terminus of the first probe is adjacent to the deleted sequence. In the case of a deletion the first probe will not include the mutation but rather will terminate at the mutation. In some embodiments, the first probe comprises a detectable moiety. In some embodiments, the first probe is devoid of a detectable moiety.

In some embodiments, the method further comprises contacting the amplification products with a second probe. In some embodiments, the second probe is complementary to the amplification product. In some embodiments, complementary is perfectly complementary. In some embodiments, the second probe and first probe are not complementary. In some embodiments, the first and second probes do not share common sequence. In some embodiments, the first and second probes are complementary to different region of the amplification products. In some embodiments, the second probe is complementary to a region of the amplification products directly adjacent to the mutation. In some embodiments, the first probe and second probe hybridize adjacent to one another to an amplification product that comprises the mutation. It will be understood by a skilled artisan that the first and second probes are designed such that they can be ligated one to the other only if an amplification product containing the mutation is present. In the case of a point mutation, a terminus of the first probe hybridizes only to the mutated nucleotide and not the wild-type nucleotide. The second probe hybridizes adjacent to the mutated nucleotide (or indeed the wild-type nucleotide). When the mutant base is present the end of the first probe is adjacent to the end of the second probe and they can be ligated. When the mutant base is absent the terminal nucleotide of the first probe doesn't hybridize and thus thought the second probe is still present the ligation reaction cannot occur due to the absence of a second terminus for ligation. In the case of a deletion mutation, the first and second probes each are adjacent to one side of the deleted region. Thus, when hybridizing to a mutant amplification product the two probes are adjacent and can ligate. In the case of a wild-type amplification product, both probes will hybridize but due to the intervening (not deleted) sequence, they cannot ligate. In the case of an insertion the first probe comprises the insertion sequence at a terminus. The first probe would terminate with the terminus of the insertion such that the second probe that is outside the insertion would be adjacent to the first probe and can ligate. In the absence of the insertion, much like the case of the point mutation, the terminus of the first probe is not hybridized and ligation cannot occur. In some embodiments, the second probe comprises a detectable moiety. In some embodiments, the second probe is devoid of a detectable moiety.

In some embodiments, the method further comprises ligating the first probe and second probe. In some embodiments, the ligation is ligation of first probe and second probe that are hybridized to an amplification product. In some embodiments, the amplification product is an amplification product comprising the mutation. In some embodiments, the ligation produces a ligated product. In some embodiments, the ligated product comprises the first and second probes. In some embodiments, the ligated product comprises a difference in length from the amplification products. In some embodiments, the ligated product comprises a difference in length from the first probe. In some embodiments, the ligated product comprises a difference in length from the second probe. In some embodiments, the ligated product comprises a difference in length from the RNA. In some embodiments, the difference in length is at least 5, 6, 7, 8, 9, 10, 11, 12, 13, 14, 15, 16, 17, 18, 19, 20, 25, 30, 35, 40,45, 50, 60, 70, 80, 90 or 100 nucleotides. Each possibility represents a separate embodiment of the invention. In some embodiments, the difference in length is at least 15 nucleotides. In some embodiments, the difference in length is at least 20 nucleotides. In some embodiments, the difference in length is at least 100 nucleotides.

In some embodiments, the identifying is identifying the ligated product as it passes through the nanopore. In some embodiments, the method further comprises identifying the ligated product as it passes through the nanopore. In some embodiments, passes through is translocates. In some embodiments, identifying as it passes through is measuring dwell time. In some embodiments, identifying as it passes through is identifying a detectable moiety on the ligated product. In some embodiments, the identifying is detecting the detectable moiety on the ligated product. In some embodiments, the probes are devoid of a detectable moiety. In some embodiments, the identifying is identifying by dwell time. In some embodiments, the identifying comprises measuring duration of a change in electrical current. In some embodiments, the duration is proportional to a length of a nucleic acid molecule translocating through the nanopore. In some embodiments, measuring the duration and associating it to a length allows for distinguishing between the ligated product and the amplification product. In some embodiments, a difference in dwell time allows for distinguishing between the ligated product and the amplification product. In some embodiments, a difference in dwell time allows for distinguishing between the ligated product and first probe. In some embodiments, a difference in dwell time allows for distinguishing between the ligated product and second probe. In some embodiments, a difference in dwell time allows for distinguishing between the ligated product and RNA.

In some embodiments, the method further comprises administering a therapeutic agent or treatment that treats the disease. In some embodiments, a therapeutic agent is administered. In some embodiments, a therapeutic treatment is administered. In some embodiments, the therapeutic agent/treatment is an agent/treatment that specifically treats the disease. In some embodiments, the administering is to a subject diagnosed with the disease. In some embodiments, the administering is to a subject that provided a sample that was identified to contain the first RNA species. In some embodiments, the administering is to a subject that provided a sample that comprised an amount of the first RNA species above a predetermined threshold. In some embodiments, the therapeutic agent is a targeted therapy and targets the gene or protein of the first RNA species. In some embodiments, the disease is a bacterial disease and the therapy is an antibiotic. In some embodiments, the disease is a viral disease and the therapy is an antiviral therapy. Antibiotics and anti-virals are well known in the art and any such therapy may be employed. Indeed, the therapy selected can be determined by a skilled physician once the subject has been correctly diagnosed. In some embodiments, the method comprises quarantining or sending to quarantine the subject that provided a sample comprising the first RNA species. In some embodiments, the therapeutic agent/treatment is an anti-proliferative agent/treatment. In some embodiments, anti-proliferative is anti-cancer. Anti-cancer agents/treatments are well known in the art and include, for example, surgery, radiation therapy, chemotherapy, and immunotherapy to name but a few.

As used herein, the term "about" when combined with a value refers to plus and minus 10% of the reference value. For example, a length of about 1000 nanometers (nm) refers to a length of 1000 nm+- 100 nm.

It is noted that as used herein and in the appended claims, the singular forms "a," "an," and "the" include plural referents unless the context clearly dictates otherwise. Thus, for example, reference to "a polynucleotide" includes a plurality of such polynucleotides and reference to "the polypeptide" includes reference to one or more polypeptides and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

In those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all subcombinations of the various embodiments and elements thereof are also specifically embraced by the present invention and are disclosed herein just as if each and every such sub-combination was individually and explicitly disclosed herein.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

Various embodiments and aspects of the present invention as delineated hereinabove and as claimed in the claims section below find experimental support in the following examples.

### EXAMPLES

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Strategies for Protein Purification and Characterization - A Laboratory Course Manual" CSHL Press (1996). Other general references are provided throughout this document.

### Materials and Methods

Sample preparation for nanopore experiments: Either DNaseI-treated RNA extracted from human cell lines or SARS-CoV-2 RNA (control 2, MN908947.3, Twist 102024.1) was reverse transcribed with specific primers (Table 1) and either subjected to second strand synthesis or to PCR amplification (see sample preparation scheme in **Figures 1A** and **2A****).** Prior to nanopore sensing experiments, the samples were either subjected to serial enzymatic digestion steps ("purification-free" sample) or purified using a commercial PCR clean-up kit.

**Table 1. Gene-specific primers used in SARS-CoV-2 experiments.**

| No | Gene | Oligo Name | Sequence (5'---3') (SEQ ID NO:) |
|---|---|---|---|
| 1 | Human RPP30 | hRPP30_For2 | AGATTTGGACCTGCGAGC (1) |
| 2 | | hRPP30_Rev5 | GACAATCTTCATCTCCTTCTGAT (2) |
| 3 | Viral SARS-CoV2 RdRp | vRdRp_For2 | GGTAACTGGTATGATTTCGGT (3) |
| 4 | | vRdRp_Rev2 | CTGGTCAAGGTTAATATAGGCATT (4) |
| 5 | Viral SARS-CoV2 RdRp | vRdRp_For3 | CTCATCAGGAGATGCCAC (5) |
| 6 | | vRdRp_Rev3 | GCAATTTTGTTACCATCAGTAGAT (6) |

Sample preparation for RT-qPCR of cell lines: Total RNA was isolated using GeneJet RNA Purification Kit (Thermo Fisher scientific), treated with DNase I (NEB) and further purified according to the manufacturer's instructions. 50 ng of DNaseI-treated RNA was reverse transcribed with random hexamers in a reaction mix (10 mM MgCl₂, 1x RT buffer, 250 µM pooled dNTPs, 1 U/µl RNAse inhibitor, and 2.5 U/µl Moloney Murine Leukemia Virus reverse transcriptase; all from Thermo Fisher Scientific) using the following procedure: incubation at 23°C for 15 min, synthesis at 42°C for 45 min, denaturation at 95°C for 5 min, and cooling at 4°C. The cDNA was amplified by qPCR using SYBR Green dye (PerfeCTa SYBR Green Fastmix, Quanta Biosciences) and primers for GOI1 or GOI2. PCR was performed under the following conditions: 95°C for 2 min followed by 40 cycles of 95°C for 7 s, 60°C for 15 s and 72°C for 20 s.

Sample preparation for RT-qNP analysis of cell lines: 50 ng total RNA was extracted from cell line 1 and 2, and reverse transcribed with GOI specific primers for each gene separately or with a combination of desired primers in a reaction mix (10 mM MgCl₂, 15 pmol of each specific primer, 1x RT buffer, 500 µM pooled dNTPs, and 200 U/µl Maxima H Minus Reverse transcriptase) at 60°C for 30 min. The reaction was terminated at 85°C for 5 min. Subsequently, cDNA was amplified to the specified PCR cycle using Kapa HiFi polymerase. The amplified cDNA was treated with 2 U RNase I (Thermo Fisher Scientific) at 37°C for 30 min. Next, the sample was treated with 4 µg ProK (Thermo Fisher Scientific) and 0.2% SDS at 37°C for 30 min.

Sample preparation for RT-qNP analysis of SARS-CoV-2 RNA and hRPP30: Either DNaseI-treated RNA, extracted from HCT116 cells, or synthetic SARS-CoV-2 RNA were reversed transcribed with specific primers (Table 1) for human RPP30 cDNA or for two amplicons within the RdRp open reading frame of SARS-CoV-2. The reaction contained 1x isothermal buffer, 6 mM MgSO4, 1.4 mM dNTPs, 0.2 µM of each primer, 3 U warmstart RTx and 6 U Bst 2.0. The reaction was carried out at 62°C for 30 min. cDNA samples were treated with 20 U of Exonuclease I (NEB) at 37°C for 15 min, followed by 4 U RNase I (Thermo Fisher Scientific) at 37°C for 30 min, and finally 0.16 U of ProK (NEB) in 0.2% SDS for 30 min.

Cell Lines: A cell lines were purchased from American Type Culture Collection (ATCC; Manassas, VA). The cells were cultured either in RPMI (cell line 1) or in DMEM (cell line 2) media supplemented with 10% fetal calf serum (FCS). These adherent cell lines were grown in flasks in a humidified incubator at 37°C with 5% CO₂ and harvested at 80% confluency for subsequent total mRNA extraction. All used cell lines were authenticated by genotyping and were confirmed to be mycoplasma-free.

Device and Nanopore Fabrication: Nanopore chips were fabricated on a 4" silicon wafer coated with silicon dioxide (SiO₂, 500 nm) and low-stress amorphous silicon nitride (SiNₓ, 50 nm). The SiNₓ was locally thinned to 8-10 nm (~2 µm circular wells) by reactive ion etching, followed by wet etching with buffered hydrofluoric acid etching to remove the SiO₂. The etched SiNₓ and SiO₂ acted as a hard mask for subsequent anisotropic Si etching in KOH (33% m/v).

Nanopore devices were cleaned in a 2:1 solution of H₂SO₄:H₂O₂ and subsequently glued using EcoFlex^{™} (smooth-on) onto a custom-made Teflon insert, immersed in buffer (1 M KCl, 40 mM Tris-HCl, 1 mM EDTA, pH 7.5), and placed in a Teflon cell. The buffer was filtered using a 0.02 µm syringe filter before use. Two Ag/AgCl pellet electrodes (A-M Systems, Sequim, WA) were connected to an Axon Axopatch 200B patch-clamp amplifier.

Nanopores were drilled in the thinned SiNₓ regions using controlled breakdown of dielectric (CBD) as previously reported. An in-house voltage/current amplifier and custom LabVIEW software (National Instruments) were used for the CBD process. Pore formation was terminated when the current exceeded a pre-set threshold, which was set to ~0.3-0.4 nA measured under 300 mV after each pulse (pulses of 8-9 V with duration of 225 ms). The pore was then expanded using alternating low voltage pulses of 1-3 V with duration of 225 ms to the desired diameter of ~4 nm, estimated according to the open pore current and the membrane properties **(****Fig. 8A-B**). The CBD profile was Weibull distributed.

Data acquisition and GMM analysis: Prior to adding the sample, the nanopores were kept under a low probing voltage (0.15 to 0.3 V) in a buffer solution (1 M KCl, 40 mM Tris-HCl, 1 mM EDTA, pH 7.5) to obtain a stable open pore current. During the experiment, translocation events were monitored using an Axon 200B amplifier, filtered at 100 kHz and acquired using a custom LabVIEW software (National Instruments). After collecting the data, offline analysis was performed using a custom LabVIEW program to extract the dwell time (*t_{D}*), current blockage (*ΔI*) and arrival time (*tₐ*) of each translocation event according to an electrical threshold.

**Figures 3E-H** describe the general data analysis routine. In short: in the first step, density histograms for each axis are generated (x: log-scale of the dwell time; y: blockage amplitude amplitude). The peak values of the blockage amplitude and the log-scale dwell time are found, and the mean and covariance matrix (half peak width) is calculated for each peak. The ratio of the peak amplitudes is used as an initial estimate of the mixing proportion. These parameters are used as initial conditions for the GMM algorithm that clusters the data into two groups. The posterior probability, which represents the likelihood that an event belongs to a specified population (GOI and RG), is calculated according to Squires, et al., "Nanopore sensing of individual transcription factors bound to DNA", Sci. Rep. 5, 1-11 (2015), herein incroporated by reference in its entirety, and presented as a distribution color-map, in which yellow dots correspond to the higher probability (>0.7) of belonging to a specific population. Data analysis was performed using MATLAB (MathWorks, Natick, MA). All graphs and corresponding fits were plotted using Igor Pro 6 (Wavemetrics, Lake Oswego, OR).

Optimizing conditions for synthesis and multiplex detection of cDNA targets: Extensive optimization was performed to achieve high specificity and yield of cDNA products for all target genes. G6PDH was selected as a reference gene for both GOIs, based on previous studies. The corresponding cDNA products were 123 bp GOI2, 360 bp GOI1 and 1231 bp G6PDH. The following optimal annealing temperatures were chosen for each cDNA: 58-70°C for GOI1, <68°C for GOI2 and <70°C for G6PDH. The sequence of the cDNA fragments was confirmed by Sanger sequencing.

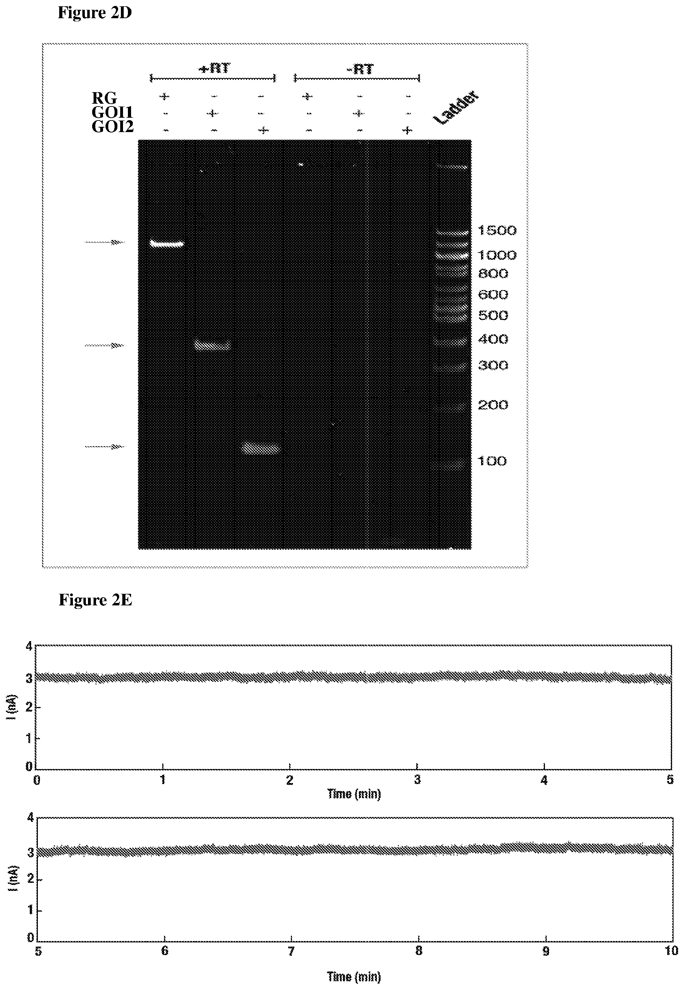

Negative control of purification-free assay and nanopore sensing: To control for translocation events arising from contaminating background molecules, such as enzymes and RNA, nanopore sensing experiments were performed on -RT samples. These samples underwent the whole process but did not contain the RT enzyme. As demonstrated in **Figure 2E****,** the current trace displayed negligible or no translocation events over extended periods of time, showing that the purification-free assay is suitable for detection of cDNA reverse transcripts.

Controls and characteristics of the cell-line samples using multiplex sensing: In all the prepared multiplexed samples from the RKO cell line, no bands were observed for either of the GOIs as expected (see **Fig. 4A****).** Hence, the RKO cell line was used as a negative control for both GOI genes, while the RG band was readily obtained, controlling for the sample preparation method.

Nanopore fabrication using CBD and characterization: The solid-state nanopores fabricated in localized thin regions using controlled dielectric breakdown (CBD) as described in Zrehen, et al., "Real-time visualization and sub-diffraction limit localization of nanometer-scale pore formation by dielectric breakdown.", Nanoscale 9, 16437-16445 (2017), and were Weibull-distributed. The membrane was ≤10 nm thick in the area where the pore was formed. Representative noise power spectral density (PSD) and current-voltage curves are shown in **Figures 8A-B**. The I-V curve **(****Fig. 8B****)** shows a symmetric and linear relationship between the current and voltage, supporting the formation of symmetric pores.

### Example 1: Method for ultrasensitive detection

To date nanopores have not been utilized for ultralow mRNA expression level quantification from cells or from clinical samples, partly due to two main factors:
i) the inherent complexity of directly processing the biological sample (i.e. cell extract, or plasma sample) containing thousands of different molecular species including proteins, lipids and nucleic acids that could either block the pore or produce erroneous signals;
ii) due to the limited selectivity of solid-state nanopores, which relies solely on the biomolecules charge and cross-section.

In contrast, the herein disclosed method called reverse transcription quantitative nanopore sensing (RT-qNP), shown schematically in **Figure 1****,** involves serial enzymatic digestion of the off-target molecules and downstream electrostatic selection of the target molecules, thus avoiding lossy purification in between steps. Total RNA is extracted either from cells or plasma obtained from CRC patients, or any other clinical source including saliva **(****Fig. 1A****).** Subsequently, it is treated with DNase I to digest genomic DNA or cell free DNA (cfDNA) present in the sample. Then specific cDNAs are synthesized by reverse transcription (RT) and the second DNA strand is co-synthesized **(****Fig. 1B****).** In some cases, target genes are then amplified by a two to five PCR cycles using specific sets of primers. This produces cDNAs of the target mRNAs, with optimal lengths for downstream nanopore analysis. Bypassing any clean-up or purification steps, we then use RNase I to digest all remaining RNAs, and Proteinase K (ProK) protease to digest proteins in the sample **(****Fig. 1C****).** The product is introduced directly to the nanopore for subsequent, label-free, analysis. Notably, all remaining enzymes in the sample preparation are electrophoretically repelled from the nanopore, whereas the negatively charged dsDNA molecules are strongly attracted to the nanopore **(****Fig. 1D****).**

### Example 2: Single-molecule mRNA quantification using solid-state nanopore biosensors

In RT-qNP total RNA is extracted either from cells or any other biological source, and all subsequent steps are additive and do not entail any purification steps **(****Fig. 1A-C**). First, complementary DNA (cDNA) is synthesized by reverse transcription (RT), followed by synthesis of the second DNA strand by a DNA polymerase. Next, RNase 1 and Proteinase K (ProK) are added in subsequent steps to digest all remaining RNA and proteins. Finally, the product is introduced directly to the nanopore for label-free analysis. Translocation of the negatively charged double-stranded cDNA through the NP leads to a distinct drop in ionic current, whereas the remaining undigested enzymes are electrophoretically repelled from the NP, and digested RNA or enzymes cause only brief current blockages that are easily identified and rejected.

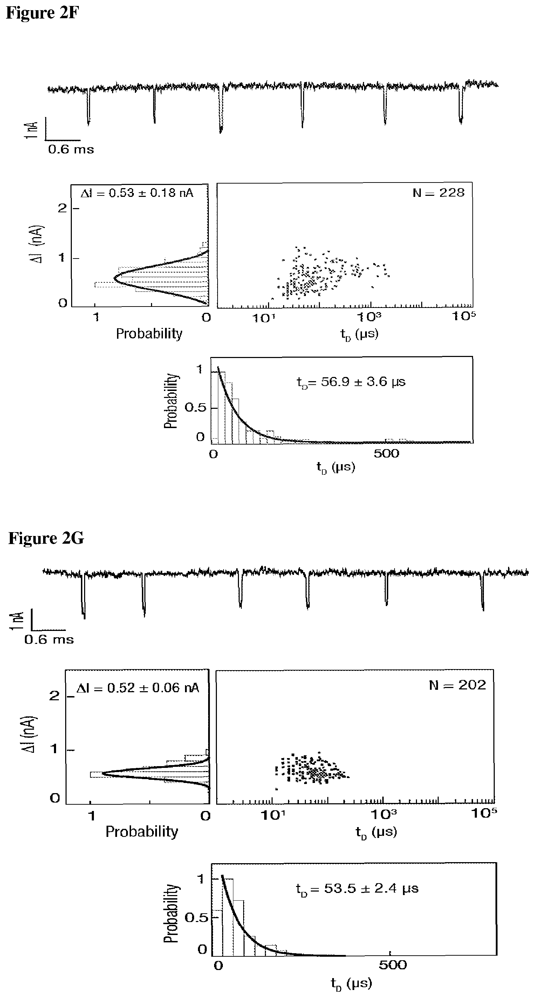

**Figure 2B** shows an experimental validation of the conversion process, in which two samples processed from the same RNA source either with or without reverse transcriptase (termed '+RT' and '-RT', respectively) are exposed to the nanopore. Each sample contained 50 ng of total RNA, and gene-specific primers to produce a 360 bp amplicon in the GOI1 open reading frame (see Materials and Methods and **Fig. 2D****).** The open-pore ionic current was stable before the sample was added. At *t =* 1 min the -RT sample was introduced to the *cis* side of the NP, resulting in a mild increase in the electrical noise, presumably due to the free digested nucleotides in the solution, however no ionic current events were observed. At *t* = 2 min the +RT sample was added, leading to clear translocation events **(****Fig. 2B****,** right panel). In a separate experiment, the open pore current was continuously recorded for an extended period of 10 minutes after the addition of -RT, confirming that no non-specific translocation events occur unless the reverse transcriptase is present **(****Fig. 2E****).** Another control experiment in which ~10³ fold more concentrated +RT product was purified using a commercial cleanup kit **(****Fig. 2F****)** showed nearly identical statistical distributions to the ones recorded using the purification-free method **(****Fig. 2G****),** indicating that the observed translocation events in the unpurified +RT sample were indeed caused by the cDNA product.

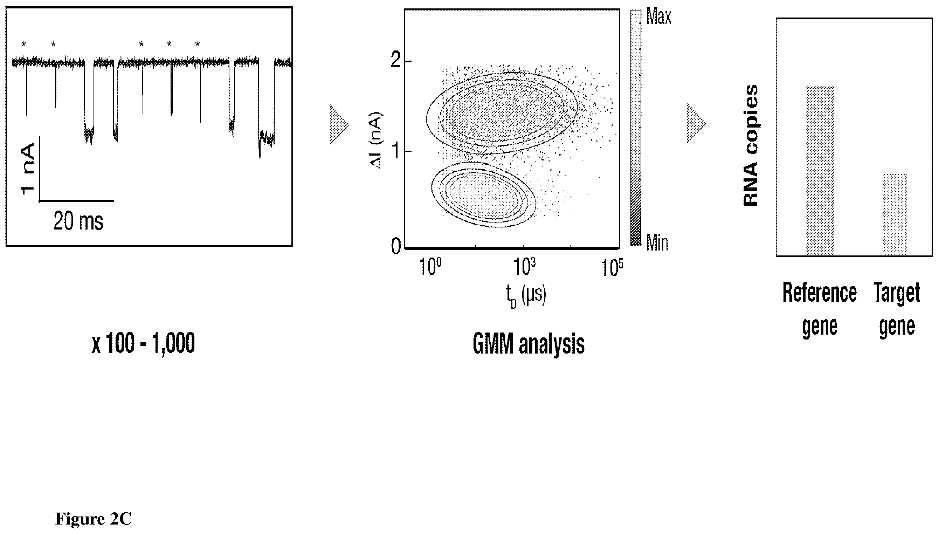

The final step of the NP sensing method is shown in **Figure 2C****.** First, translocation events were collected and analyzed based on their blockage current amplitude (Δ*I*) and dwell time (*t_{D}*)*.* A peak-finding algorithm was then applied to 2D density plots of Δ*I* vs. log (*t_{D}*) to obtain the initial conditions for a statistical analysis using a Gaussian mixed model (GMM) algorithm. The GMM efficiently resolves populations of events in a mixture containing two or three types of DNA. Between 100 to 1,000 events are typically sufficient to produce statistically robust datasets. The relative quantities of ds-cDNA reverse transcribed from different genes can then be calculated either from the total number of events in each cluster, or from the relative event rates of the populations.

### Example 3: Nanopore quantification of mixtures containing multiple cDNAs

To characterize the ability of the method to quantify relative concentrations of cDNAs in a mixture, nanopore measurements were performed using a mixture of two cDNAs, each containing a gene of interest (GOI) and a reference gene (RG). In these experiments, the cDNA was sufficiently amplified and purified using standard procedures to allow accurate quantification of the dsDNA molecules concentrations using UV-Vis spectrometry. The size of the amplicons were as follows: GOI1 was 360 bp, GOI2 was 123 bp, and RG was 1231 bp.

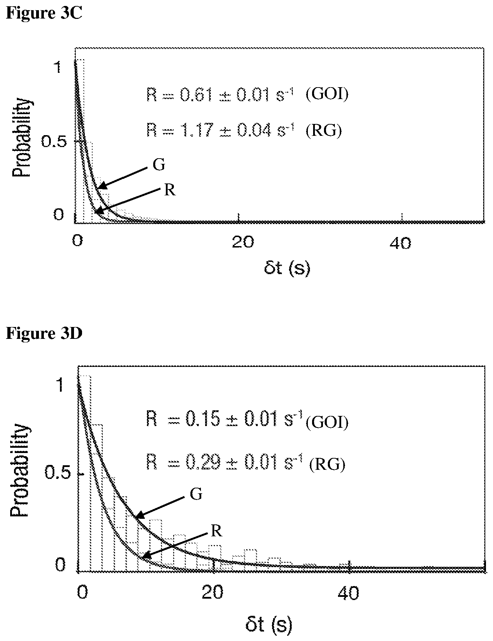

**Figure 3A** shows the event density plot and concatenated ionic current traces obtained from a mixture of 0.5 nM GOI1 and 1 nM RG. The two populations of translocation events are clearly separated in both Δ*I* and *t_{D}.* A GMM analysis of the data identifies two populations (see Materials and Methods and **Fig. 3E-H**), from which the two populations arrival time histograms were calculated **(****Fig. 3C****).** Exponential fits of the histograms yielded event rates of 0.61±0.01 s-1 and 1.17±0.04 s-1 for GOI1 and RG, respectively. The ratio of these two event rates is 0.52±0.03, which closely matches the ratio of concentrations in the mixture. Additional nanopore results from a mixture of 0.5 nM GOI2 and 1 nM RG are shown in **Figure 3B****,** yielding two distinct populations and arrival time histograms with event rates of 0.15±0.01 s-1 for GOI2 and 0.29±0.01 s-1 for RG; a factor of 0.52±0.04 **(****Fig. 3D****).**

The ratio of event rates determined by the GMM closely matches the concentration ratio of the two cDNA species in the mixture, highlighting the robustness of the method. The event classification is accurate despite slight differences in the DNA lengths and nanopore diameter between experiments, both affecting the absolute events rate measurements. It was therefore concluded that the after GMM classification based on arrival time histograms can provide a robust estimation for relative cDNA concentration, and that relative quantification against an internal reference gene can compensate for variability between pores and experiments.

### Example 4: RT-qNP analysis of mRNA expression cell lines

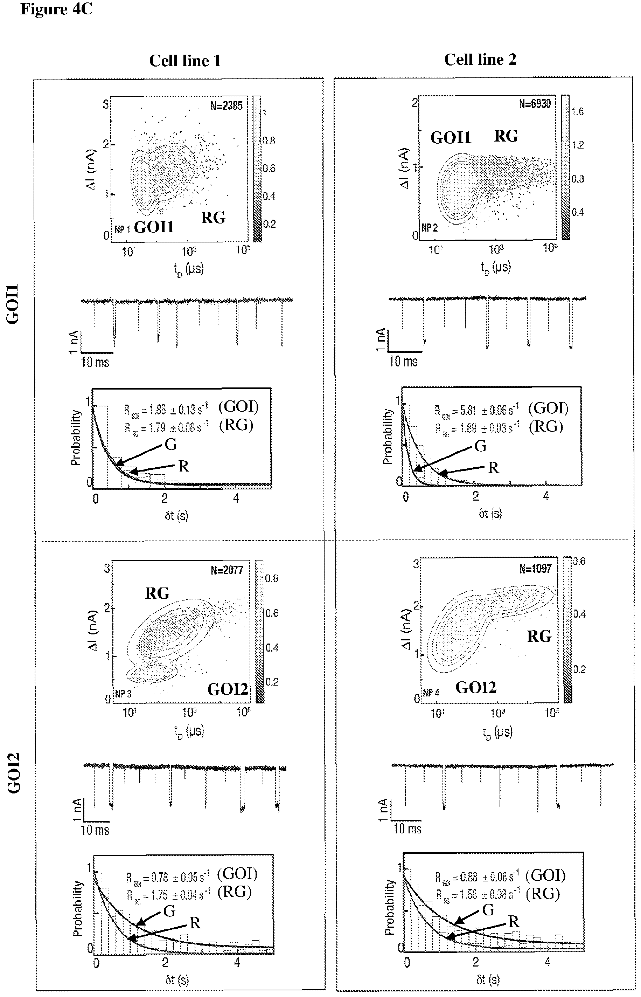

RT-qNP analysis was used to determine the mRNA expression levels of GOI1 and GOI2 relative to the reference gene (G6PDH) in two cell lines. In four separate experiments, relative populations event rates of 16-fold amplified cDNA were evaluated. Validation of multiplexed sample preparation from each cell line by gel analysis is presented in **Figure 4A** and summarized in **Figure 4B****.** Raw translocation events are shown in **Figure 4C****,** as well as the density diagrams which were used as initial predictors for GMM-based analysis. The top two panels correspond to the GOI1 analysis and the bottom panels to GOI2, as indicated. In all cases the corresponding event arrival time histograms were generated and fitted by exponential functions, and GMM-based event classification was applied.

The mean event rates for the RG, calculated from the arrival time histograms in each data set, are shown in **Figure 5A** in dark grey. Similar absolute rates (roughly 1.75 events/sec) were consistently obtained in all experiments with small differences attributed primarily to pore-to-pore variations. This indicates that G6PDH expression can be used for reliable normalization in both cell lines. The relative capture rates, shown in light grey, indicate a ~3-fold higher GOI1 mRNA content in cell line 2 as compared to cell line 1. In contrast, only a minor difference in GOI2 expression was found between the two, and the expression of this gene was lower than that of the RG.

The results from the RT-qNP method were compared to an RT-qPCR benchmark. To quantify gene expression using RT-qPCR, a calibration curve of the threshold cycle (C_{T}) for known concentrations of RNA was constructed **(****Fig. 5C****)** and it was used to determine the concentration of GOI and RG mRNA in cell line samples. The relative expression (RE) of each gene was calculated as the ratio between the GOI and RG concentrations, both normalized to the concentration of that gene in cell line 2 (see Materials and Methods). To directly compare the results, RT-qNP measurements were normalized in the same way, with events populations rates substituted for concentrations.

The results in **Figure 5B** show good agreement between the RT-qPCR and RT-qNP methods, indicating that the relative event rates accurately reflect mRNA abundance in the sample. Strikingly, however, the GOI1 expression in cell line 1 was too low to be detected using RT-qPCR. By contrast, RT-qNP sensing identified a small yet reliable quantity of GOI1 **(****Fig. 5B****,** left, dark grey bar). This result highlights the sensitivity of nanopore sensing for quantification of mRNAs at low concentrations, while maintaining specificity in the classification of translocation events based on their amplitude and dwell time.

### Example 5: RT-qNP mRNA quantification compared with RT-qPCR

To determine how ssNP sensing at low initial RNA concentrations can benefit from limited amplification, the assay was modified to include cDNA amplification starting from 16-fold (4 cycles) down to 2-fold (1 cycle). **Figure 6A** shows the results of RT-qNP sensing of GOI1 cDNA reverse transcribed from 50 ng of total RNA extracted from cell line 2. Typical continuous 3 minute ionic current traces are shown in the top panel of **Figure 6A****.** Notably, even in the extreme case of 2-fold amplification, tens of events were collected in ~20 minutes, and the GMM algorithm identified a single population with similar properties in each of the three cases, indicating that the events are indeed related to GOI1 cDNA. The extreme sensitivity of the RT-qNP sensing is clearly attributed to the sample generation process, as the inclusion of any purification steps would render the ssNP measurements impractical requiring at least ~1000-fold amplification.

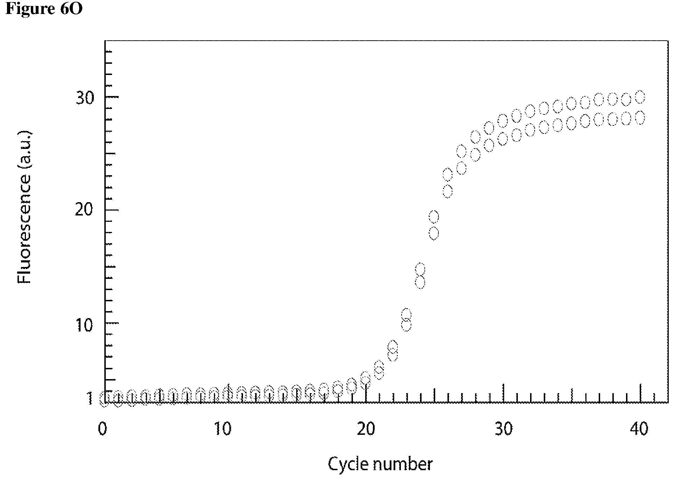

**Figure 6B** shows a direct comparison of the sensitivity of RT-qNP sensing and RT-qPCR for detecting GOI1, in terms of capture rate (for the NP) or fluorescence intensity (for RT-qPCR). qPCR was run in duplicate and averaged **(****Fig. 6O****).** The capture rate of the ssNP follows the expected exponential increase from 1-7 amplification cycles, and eventually saturates at high concentrations of cDNA as the mean inter-event time approaches the translocation dwell-time. In all cases the average event capture rate from a few tens of events to >2,000 events were evaluated. Errors were established from exponential fits to the events capture rate histograms **(****Fig. 5C****).** RT-qPCR amplification using the same 50 ng total RNA sample and primer set required ~20 amplification cycles before a detectable signal was produced. The RT-qPCR analysis shown here represents optimized PCR-primer set sensing **(****Fig. 6C-N**), indicating that the RT-qNP method provides a ~250,000-fold sensitivity improvement, as compared with RT-qPCR.

### Example 6: RT-qNP analysis of SARS-CoV-2 RNA

The COVID-19 pandemic has highlighted the importance of RNA detection as a diagnostic tool. Currently, the vast majority of nucleic acid tests are based on RT-qPCR amplification of a SARS-CoV-2 gene such as RdRp or ORF-1b. Such tests are not quantitative, and their binary diagnostic outcome is based on an empirical threshold of amplification cycles (commonly 35 or 40), rather than on quantitative RNA abundance. The arbitrary nature of the diagnostic threshold, and its variability between tests that use different primers, complicates comparisons of viral load and may lead to a large number of false positives. Quantification of viral RNA against a reference gene may provide a way to overcome these challenges.

To illustrate the flexibility of RT-qNP, the method was adapted to quantify SARS-CoV-2 viral RNA against a human reference gene (RPP30) of known concentration. While the workflow remained conceptually identical to the one shown in **Figure 1****,** the RT and cDNA synthesis was combined in a single reaction, using warmstart RTx (NEB) and Bst 2.0 (NEB) to shorten the reaction time and further improve its overall efficiency (see Materials and Methods). To increase the detection sensitivity, two sets of primers targeting the viral RNA were used, and one targeting the human RPP30 gene (see Materials and Methods). No downstream PCR amplification was required for the nanopore measurements.

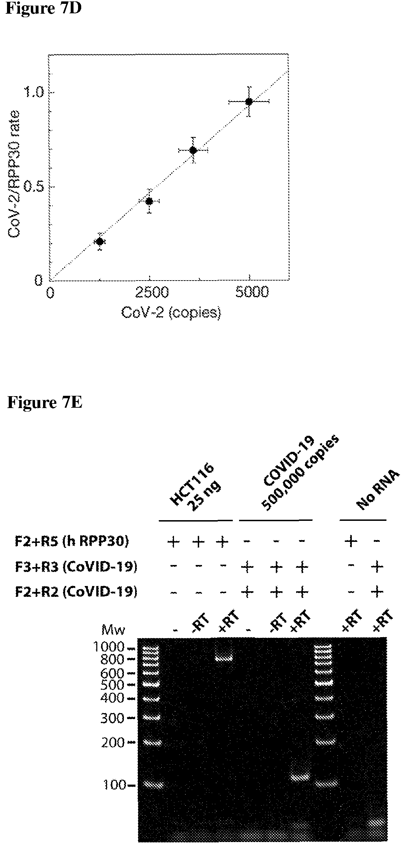

**Figures 7A-C** show nanopore results of cDNA detection after conversion from SARS-CoV-2 RNA (2,500 copies) and total human mRNA (0.25 ng) from HCT116 cell lines. The primers for the viral and human sequences respectively yielded cDNA fragments 107-108 bp and 758 bp in length **(****Fig. 7E****).** The GMM analysis successfully distinguished two populations of translocation events **(****Fig. 7B****),** and an exponential fit to the event arrival time **(****Fig. 7C****)** yielded relative event rates for the two targets.

RT-qNP quantification was performed over a clinically relevant concentration range of synthetic SARS-CoV-2 RNA (1,250-5,000 copies), mixed with RPP30 mRNA extracted from a fixed amount of 0.25 ng total human RNA from HCT116 colon cancer cell line. **Figure 7D** shows the event rate of CoV-2 relative to the co-measured RPP30 as a function of the number of RNA copies used in the upstream conversion process. Event diagrams and histograms for each of the concentrations are shown in **Figure 7F-K**. The outstanding linearity of the relative event rate across multiple pores and independently prepared samples suggests that RT-qNP can be used to quantify small changes in relative abundance. Combined with the fact that no PCR amplification is required, this result underlines the potential of single-molecule RNA detection methods for accurate quantification of viral load.

### Example 7: RT-qNP analysis of KRAS

A proof-of-concept experiment was performed to test the ability to specifically detect KRAS mutants. Probes were designed with specific sequences that target two regions along the KRAS genomic sequence (Table 2). Specifically, to target known CRC (and other cancers) mutations in KRAS, the probes were designed to end at the mutation site. The probe (66 bp) perfectly hybridizes to the KRAS genomic DNA, but the last nucleotide only hybridizes to the point mutation. This probe can further be ligated to a biotinylated probe (19 bp) to form a longer biotinylated strand using DNA ligase. The biotinylated probe hybridizes to the sequence directly on the other side of the point mutation. Ligation thus only occurs with the first probe hybridizes to the mutated base. The biotinylating is used for a purification step with magnetic streptavidin beads. Only ligated 85 bp fragments and the 19 bp biotinylated probe are pulled out. The biotinylated DNA is analyzed using the solid-state nanopore sensor. Each translocation of the longer (the ligated DNA strand) molecule is counted and considered a copy of the KRAS mutation.

**Table 2: Gene-specific primers used in KRAS experiments.**

| No | Gene | Oligo Name | Sequence (5'---3') (SEQ ID NO:) |
|---|---|---|---|
| 1 | *KRAS* WT | *KRAS_*Exon2_P1_WT | GCAAGCAGTGTCTTGCGCAAGCAGTGT CTTGCTAAGCGTACGTGCTTACACTCTT GCCTACGCCAC (7) |
| 2 | | *KRAS*_Exon2_P2_comb | pCAGCTCCAACTACCACAAG - biotin (8) |
| 3 | *KRAS* G12D | *KRAS*_Exon2_P1_G12D | GCAAGCAGTGTCTTGCGCAAGCAGTGT CTTGCTAAGCGTACGTGCTTACACTCTT GCCTACGCCAT* (9) |
| 4 | | *KRAS_*Exon2_P2_comb | pCAGCTCCAACTACCACAAG - biotin (10) |
| 5 | *KRAS* G13D | *KRAS_*Exon2_P1_G13D | CCTGACATGAATCAGGCCTGACATGAAT CAGGTAAGCGTACGTGCTTAAGGCACTC TTGCCTACGT* (11) |
| 6 | | *KRAS*_Exon2*_*P2_G13D | pCACCAGCTCCAACTACCAC - biotin (12) |
| 7 | - | Quencher | TAAGCACGTACGCTTA - Q (13) |
| 8 | - | *bit "1"* | F1 - CGTTCTGTGACGAACG - Q (14) |
| 9 | - | *bit "2"* | F2 - CCTGATTCATGTCAGG - Q (15) |

| | | | |
|---|---|---|---|
| *The point mutation is marked in bold. **p stands for phosphate group; F1-first fluorophore; F2-second fluorophore. | | | |

As can be seen in **Figure 9****,** the ligated product is produced only when the correct template is present. The ligation product, which is detectable by the nanopore, can be generated both for the G12D mutation and the G13D mutation, two well-known cancer-causing mutations. Further, the nanopore can detect the difference in length between the unligated biotinylated probe (18 bp) and the ligated products (85 bp) thus uniquely identifying mutant KRAS molecules. Alternatively, each mutation can receive a differentially colored barcode and the nanopore can detect the specific colors.

## Claims

1. A method for quantifying a first species of RNA within a sample, the method comprising:
a. receiving a sample comprising RNA and substantially devoid of DNA polymers;
b. contacting said sample with a first primer that hybridizes to said first species of RNA under conditions sufficient for reverse transcription (RT) of said first species of RNA to cDNA, thereby producing a first cDNA strand complementary to at least a portion of said species of RNA and comprising said first primer;
c. treating said sample with an RNAse enzyme thereby producing a sample comprising said first cDNA strand and substantially devoid of RNA polymers;
d. contacting said sample with a second primer that hybridizes to said first cDNA strand and performing 1-5 cycles of amplification to produce amplification products with a first termini that is identical to a sequence of said first primer and a second termini that is a reverse complement to a sequence of said second primer or with a first termini that is a reverse complement of a sequence of said first primer and a second termini that is identical to a sequence of said second primer;
e. treating said sample with a proteinase enzyme, wherein said enzyme comprises a net positive charge, to produce a solution substantially devoid of polypeptides other than said proteinase;
f. depositing said solution within a first reservoir of a nanopore containing apparatus and passing said amplification products through said nanopore by running electrical current from said first reservoir to a second reservoir via said nanopore; and
g. identifying an amplification product derived from said first RNA species as it passes through said nanopore by said amplification product's dwell time within said nanopore;
wherein said method is devoid of a washing or isolation step after step (a),
thereby quantifying a species of RNA within a sample.

2. The method of claim 1, wherein said receiving comprises receiving a sample of isolated RNA or receiving a cell lysate contacted with a DNAse enzyme.

3. The method of claim 1 or 2, wherein (b) comprises contacting said sample with a reverse transcriptase and DNA nucleotides.

4. The method of any one of claims 1 to 3, wherein said step (c) occurs before said step (d) or occurs after said step (d) and said RNAse treatment produces a sample comprising said first cDNA strand and said amplification products are substantially devoid of RNA.

5. The method of any one of claims 1 to 4, wherein:
a. an enzyme carrying a net positive charge is an enzyme that when in a solution through which electrical current is passed migrates towards a negative pole;
b. said treating with a proteinase is under conditions sufficient for degradation of polypeptides to single amino acids;
c. said treating with a proteinase is under conditions sufficient for protection of said proteinase from autolysis;
d. said proteinase is proteinase K; or
e. a combination thereof.

6. The method of any one of claims 1 to 5, wherein said amplification product is:
a. at least 20 nucleotides shorter than said first cDNA strand;
b. not longer than 10,000 nucleotides; or
c. both.

7. The method of any one of claims 1 to 6, wherein said first primer, said second primer or both are DNA primers, are specific to said RNA species, are 100% complementary to said RNA species or a combination thereof.

8. The method of any one of claims 1 to 7, comprising a single cycle of amplification thereby producing on average a single amplification product for each molecule of said species of RNA.

9. The method of any one of claims 1 to 8, wherein said amplification does not comprises integrating a detectable moiety into said amplification products.

10. The method of any one of claims 1 to 9, wherein said identifying comprises:
a. detecting a change in electrical current through said nanopore;
b. detecting a change in electrical current through said nanopore duration of said change in electrical current, and wherein said duration is proportional to the length of said amplification product;
c. differentiating said amplification product derived from said RNA from at least one of a free DNA nucleotide, a free RNA nucleotide, a free amino acid, a first cDNA strand and an off-target amplification product by dwell time within said nanopore; or
d. a combination thereof.

11. The method of any one of claims 1 to 10, further comprising in step (b) contacting said sample with a third primer that hybridizes to a second species of RNA, thereby producing a first cDNA strand complementary to at least a portion of said second species of RNA and comprising said second primer; and further comprising in step (d) contacting said sample with a fourth primer that hybridizes to said first cDNA strand complementary to at least a portion of said second species of RNA to produce amplification products with a first termini that is identical to a sequence of said third primer and a second termini that is a reverse complement to a sequence of said fourth primer or with a first termini that is a reverse complement of a sequence of said third primer and a second termini that is identical to a sequence of said fourth primer, optionally wherein amplification products derived from said first species and amplification products derived from said second species differ in length by at least 20 nucleotides and are differentiated by a difference in dwell time or wherein said method comprises quantifying at least three species of RNA within said sample, wherein each amplification product derived from an RNA species is at least 20 nucleotides in length different than any amplification product derived from a different RNA species.

12. The method of any one of claims 1 to 11, wherein said first species of RNA comprises a point mutation and further comprising:
a. contacting the amplification products with a first probe perfectly complementary to said amplification products and comprising a terminal nucleotide complementary to said point mutation and a second probe perfectly complementary to said amplification products directly adjacent to said point mutation and not complementary to the same region as said first probe;
b. ligating said first probe and said second probe to produce a ligated product, such that said ligated product comprises a difference in length from said amplification products, said first probe and said second probe of at least 15 nucleotides; and
c. identifying said ligated product as it passes through said nanopore; optionally wherein said second probe comprises a detectable moiety and said identifying comprises detecting said detectable moiety as it passes through said nanopore; or wherein said second probe does not comprise a detectable moiety and said identification by dwell time comprise measuring duration of a change in electrical current, and wherein said duration is proportional to the length of a nucleic acid molecule translocating through said nanopore, allowing for distinguishing said ligated product from said amplification product.

13. The method of any one of claims 1 to 12, wherein:
a. said first species of RNA is RNA of a target gene, RNA of a disease-associated gene or RNA of a gene whose expression is indicative of the presence of a disease in said sample;
b. said second species of RNA is RNA of a control gene;
c. said second species of RNA is RNA of a gene selected from glucose-6-phosphate dehydrogenase (G6PDH) and Ribonuclease P/MRP subunit P30 (RPP30); or
d. a combination thereof.

14. A method of diagnosing a disease in a subject in need thereof, the method comprising performing the method of any one of claims 1 to 13, wherein said sample is from said subject, said first RNA species is an mRNA of a gene associated with said disease, and detection of said first RNA species in said sample indicates said subject suffers from said disease.

15. The method of claim 14, wherein at least one of:
a. detection of said first RNA species above a predetermined threshold indicates said subject suffers from said disease;
b. said disease is an infectious disease and said first species of RNA is an RNA of an infectious agent;
c. said disease is an infectious disease and said first species of RNA is an RNA of an infectious agent and said second RNA species is an RNA of a host cell infected by said infectious agent;
d. said disease is an infectious disease and said first species of RNA is an RNA of an infectious agent and said infectious disease is SARS-CoV-2 and said first RNA species is an RNA-dependent RNA polymerase (RdRP) gene mRNA;
e. said disease is **characterized by** the presence of a mutation and said first species of RNA is an RNA comprising said mutation; and
f. said disease is a proliferative disease **characterized by** the presence of a pro-proliferative or antiapoptotic mutation and said first species of RNA is an RNA comprising said mutation.

## Patentansprüche

1. Verfahren zur Quantifizierung einer ersten RNA-Spezies in einer Probe, wobei das Verfahren umfasst:
a. Empfangen einer Probe, die RNA enthält und im Wesentlichen frei von DNA-Polymeren ist;
b. Inkontaktbringen der Probe mit einem ersten Primer, der unter Bedingungen, die für die reverse Transkription (RT) der ersten RNA-Spezies zu cDNA ausreichend sind, mit der ersten RNA-Spezies hybridisiert, wodurch ein erster cDNA-Strang erzeugt wird, der zu mindestens einem Teil der RNA-Spezies komplementär ist und den ersten Primer umfasst;
c. Behandeln der Probe mit einem RNAse-Enzym, wodurch eine Probe erzeugt wird, die den ersten cDNA-Strang umfasst und im Wesentlichen frei von RNA-Polymeren ist;
d. Inkontaktbringen der Probe mit einem zweiten Primer, der an den ersten cDNA-Strang hybridisiert, und Durchführen von 1 bis 5 Amplifikationszyklen, um Amplifikationsprodukte mit einem ersten Terminus, der mit einer Sequenz des ersten Primers identisch ist, und einem zweiten Terminus, der ein reverses Komplement zu einer Sequenz des zweiten Primers ist, oder mit einem ersten Terminus, der ein reverses Komplement zu einer Sequenz des ersten Primers ist, und einem zweiten Terminus, der mit einer Sequenz des zweiten Primers identisch ist, herzustellen;
e. Behandeln der Probe mit einem Proteaseenzym, wobei das Enzym eine positive Nettoladung aufweist, um eine Lösung zu erzeugen, die von anderen Polypeptiden als der Protease im Wesentlichen frei ist;
f. Einbringen der Lösung in ein erstes Reservoir einer Nanoporen enthaltenden Vorrichtung und Durchleiten der Amplifikationsprodukte durch die Nanopore, indem elektrischer Strom vom ersten Reservoir über die Nanopore zu einem zweiten Reservoir geleitet wird; und
g. Identifizieren eines von der ersten RNA-Spezies herrührenden Amplifikationsprodukts, während es durch die Nanopore fließt, anhand der Verweildauer des Amplifikationsprodukts in der Nanopore;
wobei das Verfahren nach Schritt (a) keinen Wasch- oder Isolierungsschritt umfasst,
wodurch eine RNA-Spezies in einer Probe quantifiziert wird.

2. Verfahren nach Anspruch 1, bei dem das Empfangen das Empfangen einer Probe isolierter RNA oder das Empfangen eines Zelllysats umfasst, das mit einem DNAse-Enzym in Kontakt gebracht wurde.

3. Verfahren nach Anspruch 1 oder 2, bei dem (b) das Inkontaktbringen der Probe mit einer reversen Transkriptase und DNA-Nukleotiden umfasst.

4. Verfahren nach einem der Ansprüche 1 bis 3, bei dem Schritt (c) vor Schritt (d) oder nach Schritt (d) erfolgt und die RNAse-Behandlung eine Probe erzeugt, die den ersten cDNA-Strang umfasst, und die Amplifikationsprodukte im Wesentlichen frei von RNA sind.

5. Verfahren nach einem der Ansprüche 1 bis 4, bei dem:
a. ein Enzym, das eine positive Nettoladung trägt, ein Enzym ist, das in einer Lösung, durch die elektrischer Strom fließt, in Richtung eines negativen Pols wandert;
b. die Behandlung mit einer Protease unter Bedingungen erfolgt, die für den Abbau von Polypeptiden zu einzelnen Aminosäuren ausreichend sind;
c. die Behandlung mit einer Protease unter Bedingungen erfolgt, die für den Schutz der Protease vor Autolyse ausreichend sind;
d. die Protease Protease K ist; oder
e. eine Kombination davon.

6. Verfahren nach einem der Ansprüche 1 bis 5, bei dem das Amplifikationsprodukt
a. mindestens 20 Nukleotide kürzer als der erste cDNA-Strang ist;
b. nicht länger als 10.000 Nukleotide ist; oder
c. beides.

7. Verfahren nach einem der Ansprüche 1 bis 6, bei dem der erste Primer, der zweite Primer oder beide DNA-Primer sind, für die RNA-Spezies spezifisch sind, zu 100 % komplementär zu der RNA-Spezies sind oder eine Kombination davon.

8. Verfahren nach einem der Ansprüche 1 bis 7, umfassend einen einzigen Amplifikationszyklus, wodurch im Durchschnitt zu jedem Molekül der RNA-Spezies ein einziges Amplifikationsprodukt erzeugt wird.

9. Verfahren nach einem der Ansprüche 1 bis 8, bei dem die Amplifikation keine Integration einer nachweisbaren Einheit in die Amplifikationsprodukte umfasst.

10. Verfahren nach einem der Ansprüche 1 bis 9, bei dem das Identifizieren:
a. Erfassen einer Änderung des elektrischen Stroms durch die Nanopore;
b. Erfassen einer Änderung des elektrischen Stroms durch die Nanopore Dauer der Änderung des elektrischen Stroms, wobei die Dauer proportional zur Länge des Amplifikationsprodukts ist;
c. Unterscheiden des aus der RNA abgeleiteten Amplifikationsprodukts von mindestens einem freien DNA-Nukleotid, einem freien RNA-Nukleotid, einer freien Aminosäure, einem ersten cDNA-Strang und einem Off-Target-Amplifikationsprodukt anhand der Verweildauer in der Nanopore; oder
d. eine Kombination davon
umfasst.

11. Verfahren nach einem der Ansprüche 1 bis 10, das ferner in Schritt (b) das Inkontaktbringen der Probe mit einem dritten Primer umfasst, der mit einer zweiten RNA-Spezies hybridisiert, wodurch ein erster cDNA-Strang erzeugt wird, der zu mindestens einem Teil der zweiten RNA-Spezies komplementär ist und den zweiten Primer umfasst; und das ferner in Schritt (d) das Inkontaktbringen der Probe mit einem vierten Primer umfasst, der an den zu mindestens einem Teil der zweiten RNA-Spezies komplementären ersten cDNA-Strang hybridisiert, um Amplifikationsprodukte mit einem ersten Terminus, der mit einer Sequenz des dritten Primers identisch ist, und einem zweiten Terminus, der ein reverses Komplement zu einer Sequenz des vierten Primers ist, oder mit einem ersten Terminus, der ein reverses Komplement zu einer Sequenz des dritten Primers ist, und einem zweiten Terminus, der mit einer Sequenz des vierten Primers identisch ist, herzustellen, wobei optional die von der ersten Spezies abgeleiteten Amplifikationsprodukte und die von der zweiten Spezies abgeleiteten Amplifikationsprodukte sich in ihrer Länge um mindestens 20 Nukleotide unterscheiden und durch eine Differenz in der Verweildauer unterschieden werden, oder wobei das Verfahren das Quantifizieren von mindestens drei Spezies von RNA innerhalb der Probe umfasst, wobei jedes von einer RNA-Spezies abgeleitete Amplifikationsprodukt sich in seiner Länge um mindestens 20 Nukleotide von jedem von einer anderen RNA-Spezies abgeleiteten Amplifikationsprodukt unterscheidet.

12. Verfahren nach einem der Ansprüche 1 bis 11, bei dem die erste RNA-Spezies eine Punktmutation umfasst, und das ferner umfasst:
a. Inkontaktbringen der Amplifikationsprodukte mit einer ersten Sonde, die zu den Amplifikationsprodukten perfekt komplementär ist und ein terminales Nukleotid umfasst, das zu der Punktmutation komplementär ist, und einer zweiten Sonde, die zu den Amplifikationsprodukten direkt neben der Punktmutation perfekt komplementär ist und nicht zu derselben Region wie die erste Sonde komplementär ist;
b. Ligieren der ersten Sonde und der zweiten Sonde, um ein ligiertes Produkt zu erzeugen, so dass das ligierte Produkt eine Längendifferenz von mindestens 15 Nukleotiden zu den Amplifikationsprodukten, der ersten Sonde und der zweiten Sonde aufweist; und
c. Identifizieren des ligierten Produkts, während es die Nanopore durchläuft;
wobei optional die zweite Sonde eine nachweisbare Einheit umfasst und das Identifizieren das Nachweisen der nachweisbaren Einheit umfasst, wenn diese die Nanopore durchläuft; oder wobei die zweite Sonde keine nachweisbare Einheit umfasst und das Identifizieren anhand der Verweildauer das Messen der Dauer einer Änderung des elektrischen Stroms umfasst, wobei die Dauer proportional zur Länge eines Nukleinsäuremoleküls ist, das durch die Nanopore transloziert,
wodurch das ligierte Produkt von dem Amplifikationsprodukt unterschieden werden kann.

13. Verfahren nach einem der Ansprüche 1 bis 12, bei dem:
a. die erste RNA-Spezies RNA eines Zielgens, RNA eines krankheitsassoziierten Gens oder RNA eines Gens ist, dessen Expression auf das Vorliegen einer Krankheit in der Probe hinweist;
b. die zweite RNA-Spezies RNA eines Kontrollgens ist;
c. die zweite RNA-Spezies RNA eines Gens ist, das aus Glucose-6-phosphat-Dehydrogenase (G6PDH) und Ribonuklease P/MRP-Untereinheit P30 (RPP30) ausgewählt ist; oder
d. eine Kombination davon.

14. Verfahren zum Diagnostizieren einer Krankheit bei einem Patienten, der dies benötigt, wobei das Verfahren die Durchführung des Verfahrens nach einem der Ansprüche 1 bis 13 umfasst, wobei die Probe von dem Patienten stammt, die erste RNA-Spezies eine mRNA eines mit der Krankheit assoziierten Gens ist und der Nachweis der ersten RNA-Spezies in der Probe anzeigt, dass der Patient an der Krankheit leidet.

15. Verfahren nach Anspruch 14, bei dem:
a. der Nachweis der ersten RNA-Spezies oberhalb eines vorbestimmten Schwellenwerts anzeigt, dass die Person an der Krankheit leidet;
b. die Krankheit eine Infektionskrankheit ist und die erste RNA-Spezies eine RNA eines Infektionserregers ist;
c. die Krankheit eine Infektionskrankheit ist und die erste RNA-Spezies eine RNA eines Infektionserregers ist und die zweite RNA-Spezies eine RNA einer durch den Infektionserreger infizierten Wirtszelle ist;
d. die Krankheit eine Infektionskrankheit ist und die erste RNA-Spezies eine RNA eines Infektionserregers ist und die Infektionskrankheit SARS-CoV-2 ist und die erste RNA-Spezies eine RNA-abhängige RNA-Polymerase (RdRP)-Gen-mRNA ist;
e. die Krankheit durch das Vorhandensein einer Mutation gekennzeichnet ist, und die erste RNA-Spezies eine RNA ist, die die Mutation umfasst; und
f. die Krankheit eine proliferative Krankheit ist, die durch das Vorhandensein einer pro-proliferativen oder antiapoptotischen Mutation gekennzeichnet ist, und die erste RNA-Spezies eine RNA ist, die die Mutation umfasst.

## Revendications

1. Procédé de quantification d'une première espèce d'ARN dans un échantillon, le procédé comportant :
a. la réception d'un échantillon comportant de l'ARN et sensiblement dépourvu de polymères d'ADN ;
b. la mise en contact dudit échantillon avec une première amorce qui s'hybride à ladite première espèce d'ARN dans des conditions suffisantes pour la transcription inverse (RT) de ladite première espèce d'ARN en ADNc, produisant ainsi un premier brin d'ADNc complémentaire d'au moins une partie de ladite espèce d'ARN et comportant ladite première amorce ;
c. le traitement dudit échantillon avec une enzyme RNAse, produisant ainsi un échantillon comportant ledit premier brin d'ADNc et sensiblement dépourvu de polymères d'ARN ;
d. la mise en contact dudit échantillon avec une deuxième amorce qui s'hybride audit premier brin d'ADNc et la réalisation de 1 à 5 cycles d'amplification pour produire des produits d'amplification avec une première extrémité terminale qui est identique à une séquence de ladite première amorce et une deuxième extrémité terminale qui est un complément inverse d'une séquence de ladite deuxième amorce ou avec une première extrémité terminale qui est un complément inverse d'une séquence de ladite première amorce et une deuxième extrémité terminale qui est identique à une séquence de ladite deuxième amorce ;
e. le traitement dudit échantillon avec une enzyme protéinase, dans lequel ladite enzyme comporte une charge positive nette, pour produire une solution sensiblement dépourvue de polypeptides autres que ladite protéinase ;
f. le dépôt de ladite solution dans un premier réservoir d'un appareil contenant un nanopore et le passage desdits produits d'amplification à travers ledit nanopore en faisant circuler un courant électrique dudit premier réservoir vers un deuxième réservoir via ledit nanopore ; et
g. l'identification d'un produit d'amplification dérivé de ladite première espèce d'ARN au fur et à mesure qu'il passe à travers ledit nanopore selon un temps de séjour dudit produit d'amplification dans ledit nanopore ;
dans lequel ledit procédé est dépourvu d'une étape de lavage ou d'isolation après l'étape (a), quantifiant ainsi une espèce d'ARN dans un échantillon.

2. Procédé selon la revendication 1, dans lequel ladite réception comporte la réception d'un échantillon d'ARN isolé ou la réception d'un lysat cellulaire en contact avec une enzyme DNAse.

3. Procédé selon la revendication 1 ou 2, dans lequel (b) comporte la mise en contact dudit échantillon avec une transcriptase inverse et des nucléotides d'ADN.

4. Procédé selon l'une quelconque des revendications 1 à 3, dans lequel ladite étape (c) se produit avant ladite étape (d) ou se produit après ladite étape (d) et ledit traitement à la RNAse produit un échantillon comportant ledit premier brin d'ADNc et lesdits produits d'amplification sont sensiblement dépourvus d'ARN.

5. Procédé selon l'une quelconque des revendications 1 à 4, dans lequel :
a. une enzyme portant une charge positive nette est une enzyme qui, lorsqu'elle se trouve dans une solution à travers laquelle passe un courant électrique, migre vers un pôle négatif ;
b. ledit traitement avec une protéinase a lieu dans des conditions suffisantes pour la dégradation de polypeptides en acides aminés simples ;
c. ledit traitement avec une protéinase a lieu dans des conditions suffisantes pour protéger ladite protéinase de l'autolyse ;
d. ladite protéinase est la protéinase K ; ou
e. une combinaison de ces caractéristiques.

6. Procédé selon l'une quelconque des revendications 1 à 5, dans lequel ledit produit d'amplification est :
a. plus court d'au moins 20 nucléotides par rapport audit premier brin d'ADNc ;
b. d'une longueur ne dépassant pas 10 000 nucléotides ; ou
c. les deux.

7. Procédé selon l'une quelconque des revendications 1 à 6, dans lequel ladite première amorce, ladite deuxième amorce ou les deux sont des amorces d'ADN, sont spécifiques à ladite espèce d'ARN, sont 100 % complémentaires de ladite espèce d'ARN ou une combinaison de ces caractéristiques.

8. Procédé selon l'une quelconque des revendications 1 à 7, comportant un seul cycle d'amplification, produisant ainsi en moyenne un seul produit d'amplification pour chaque molécule de ladite espèce d'ARN.

9. Procédé selon l'une quelconque des revendications 1 à 8, dans lequel ladite amplification ne comporte pas l'intégration d'une fraction détectable dans lesdits produits d'amplification.

10. Procédé selon l'une quelconque des revendications 1 à 9, dans lequel ladite identification comporte :
a. la détection d'un changement de courant électrique à travers ledit nanopore ;
b. la détection d'une durée de changement de courant électrique à travers ledit nanopore dudit changement de courant électrique, et dans lequel ladite durée est proportionnelle à la longueur dudit produit d'amplification ;
c. la différenciation dudit produit d'amplification dérivé dudit ARN d'au moins l'un parmi un nucléotide d'ADN libre, un nucléotide d'ARN libre, un acide aminé libre, un premier brin d'ADNc et un produit d'amplification hors cible selon un temps de séjour dans ledit nanopore ; ou
d. une combinaison de ces opérations.

11. Procédé selon l'une quelconque des revendications 1 à 10, comportant en outre, à l'étape (b), la mise en contact dudit échantillon avec une troisième amorce qui s'hybride à une deuxième espèce d'ARN, produisant ainsi un premier brin d'ADNc complémentaire d'au moins une partie de ladite deuxième espèce d'ARN et comportant ladite deuxième amorce ; et comportant en outre, à l'étape (d), la mise en contact dudit échantillon avec une quatrième amorce qui s'hybride audit premier brin d'ADNc complémentaire d'au moins une partie de ladite deuxième espèce d'ARN pour produire des produits d'amplification avec une première extrémité terminale qui est identique à une séquence de ladite troisième amorce et une deuxième extrémité terminale qui est un complément inverse d'une séquence de ladite quatrième amorce ou avec une première extrémité terminale qui est un complément inverse d'une séquence de ladite troisième amorce et une deuxième extrémité terminale qui est identique à une séquence de ladite quatrième amorce, facultativement dans lequel des produits d'amplification dérivés de ladite première espèce et des produits d'amplification dérivés de ladite deuxième espèce présentent une différence de longueur d'au moins 20 nucléotides et sont différenciés par une différence de temps de séjour ou dans lequel ledit procédé comporte la quantification d'au moins trois espèces d'ARN dans ledit échantillon, dans lequel chaque produit d'amplification dérivé d'une espèce d'ARN présente une différence de longueur d'au moins 20 nucléotides par rapport à tout produit d'amplification dérivé d'une espèce d'ARN différente.

12. Procédé selon l'une quelconque des revendications 1 à 11, dans lequel ladite première espèce d'ARN comporte une mutation ponctuelle et comportant en outre :
a. la mise en contact des produits d'amplification avec une première sonde parfaitement complémentaire desdits produits d'amplification et comportant un nucléotide terminal complémentaire de ladite mutation ponctuelle et une deuxième sonde parfaitement complémentaire desdits produits d'amplification directement adjacente à ladite mutation ponctuelle et non complémentaire de la même région que ladite première sonde ;
b. la ligature de ladite première sonde et de ladite deuxième sonde pour produire un produit ligaturé, de sorte que ledit produit ligaturé comporte une différence de longueur par rapport auxdits produits d'amplification, à ladite première sonde et à ladite deuxième sonde d'au moins 15 nucléotides ; et
c. l'identification dudit produit ligaturé au fur et à mesure qu'il passe à travers ledit nanopore ; facultativement dans lequel ladite deuxième sonde comporte une fraction détectable et ladite identification comporte la détection de ladite fraction détectable au fur et à mesure qu'elle passe à travers ledit nanopore ; ou dans lequel ladite deuxième sonde ne comporte pas de fraction détectable et ladite identification selon un temps de séjour comporte une durée de mesure d'un changement de courant électrique, et dans lequel ladite durée est proportionnelle à la longueur d'une molécule d'acide nucléique transloquée à travers ledit nanopore, permettant de distinguer ledit produit ligaturé dudit produit d'amplification.

13. Procédé selon l'une quelconque des revendications 1 à 12, dans lequel :
a. ladite première espèce d'ARN est l'ARN d'un gène cible, l'ARN d'un gène associé à une maladie ou l'ARN d'un gène dont l'expression indique la présence d'une maladie dans ledit échantillon ;
b. ladite deuxième espèce d'ARN est l'ARN d'un gène de contrôle ;
c. ladite deuxième espèce d'ARN est l'ARN d'un gène choisi parmi la glucose-6-phosphate déshydrogénase (G6PDH) et la sous-unité P30 de la ribonucléase P/MRP (RPP30) ; ou
d. une combinaison de ces caractéristiques.

14. Procédé de diagnostic d'une maladie chez un sujet en ayant besoin, le procédé comportant la réalisation du procédé selon l'une quelconque des revendications 1 à 13, dans lequel ledit échantillon provient dudit sujet, ladite première espèce d'ARN est un ARNm d'un gène associé à ladite maladie, et la détection de ladite première espèce d'ARN dans ledit échantillon indique que ledit sujet souffre de ladite maladie.

15. Procédé selon la revendication 14, dans lequel au moins l'une des caractéristiques suivantes est identifiée :
a. la détection de ladite première espèce d'ARN au-dessus d'un seuil prédéterminé indique que ledit sujet souffre de ladite maladie ;
b. ladite maladie est une maladie infectieuse et ladite première espèce d'ARN est un ARN d'un agent infectieux ;
c. ladite maladie est une maladie infectieuse et ladite première espèce d'ARN est un ARN d'un agent infectieux et ladite deuxième espèce d'ARN est un ARN d'une cellule hôte infectée par ledit agent infectieux ;
d. ladite maladie est une maladie infectieuse et ladite première espèce d'ARN est un ARN d'un agent infectieux et ladite maladie infectieuse est le SARS-CoV-2 et ladite première espèce d'ARN est un ARNm de gène d'ARN polymérase (RdRP) ARN-dépendante ;
e. ladite maladie est **caractérisée par** la présence d'une mutation et ladite première espèce d'ARN est un ARN comportant ladite mutation ; et
f. ladite maladie est une maladie proliférative **caractérisée par** la présence d'une mutation pro-proliférative ou antiapoptotique et ladite première espèce d'ARN est un ARN comportant ladite mutation.
